# EUROPEAN PATENT APPLICATION

(11) **EP 4 566 590 A1**
(43) Date of publication of application: **11.06.2025**
(21) Application number: 23849498.3
(22) Date of filing: 03.08.2023
(51) Int. Cl.: A61K 9/16, A61K 9/20, A61K 9/48, A61K 47/38, A61K 47/32

(54) **TASTE-MASKING MICROSPHERE, PREPARATION PROCESS THEREFOR, AND USE THEREOF**

(30) Priority: 05.08.2022 CN 202210938199; 21.07.2023 CN 202310907725
(71) Applicant: Harbin Kanon Pharmaceutical Co., Ltd, Harbin, Heilongjiang 150500 (CN); Beijing Zhenglong Pharmaceutical Research Institute Co., Ltd, Beijing 100176 (CN)
(72) Inventor: MU, Bin, Beijing 100176 (CN); ZHAO, YuXin, Beijing 100176 (CN)
(74) Representative: CH Kilger Anwaltspartnerschaft mbB
(86) International application number: PCT/CN2023/111002
(87) International publication number: WO 2024/027794

(57) **Abstract**

The present disclosure provides a taste-masking microsphere, a preparation process therefor, and use thereof, and relates to the technical field of pharmaceutical formulations. The taste-masking microsphere comprises the following components in parts by weight: 1.5-21.2 parts of a medicament ingredient, 35-110 parts of a medicament carrier, 0-30 parts of an alkalizing agent, and 0-8 parts of a plasticizer. The taste-masking microsphere has the advantages of a good taste-masking effect and wide adaptability.

## Description

### Technical Field

The present disclosure relates to the technical field of pharmaceutical formulations, and in particular to a taste-masking microsphere, a preparation process therefor and the use thereof.

### Background Art

Vonoprazan fumarate is a novel reversible proton pump inhibitor (RPPI) and a potassium-competitive acid blocker (P-CAB). Its clinical mechanism of action is that the H' produced by the hydrolytic dissociation of vonoprazan fumarate is actively transported into the lumen of the gastric canaliculus through the action of H⁺/K⁺-ATPase on the secretory canalicular membrane of gastric parietal cells, thereby continuously blocking the newly synthesized H⁺/K⁺-ATPase and in turn producing a long-lasting acid-suppression effect. Vonoprazan fumarate is mainly used for the treatment of gastric ulcer, duodenal ulcer, erosive esophagitis and the eradicative treatment of *Helicobacter pylori.*

Meanwhile, vonoprazan fumarate itself has an intense bitter taste and poor patient compliance, limiting the clinical application thereof. In addition, the clinical application of other medicaments with intense bitter taste is also limited.

Currently, the taste-masking technologies for oral pharmaceuticals include micropellet coating technique, macroporous resin adsorption technique, taste modulation technique, microsphere technique, etc. For instance, (1) Chinese Patent Application CN 112353802 A discloses a taste-masking composition of vonoprazan fumarate, which is prepared from vonoprazan fumarate, a medicament API, and an acidic cation exchange resin by means of static exchange; (2) Chinese Patent Applications CN 102512389 A and CN 1592622 A disclose preparing medicament-containing granules into micropellets by means of fluidized bed coating using Eudragit resins; (3) Chinese Patent Application CN 101822646 A discloses adding a flavoring agent to mask the bitter taste of medicaments; (4) Chinese Patent Application CN 102958515 A discloses preparing microspheres using a phase separation method, with ethylcellulose dissolved using an organic solvent cyclohexane, and polyethylene as a phase inducer being added; (5) Chinese Patent Application CN 101836965 A discloses using Eudragit as a carrier material and forming a solid dispersion by means of solvent evaporation to prepare medicament-encapsulated microspheres; and (6) Chinese Patent Application CN 101065116 A discloses preparing polypeptide microspheres by means of phase separation, with a PLGA polymer as a carrier.

The disadvantages of the micropellet coating technique are that the operation is complicated, and since the particle size of the micropellets is 300-1200 microns, the taste is poor (with a strong gritty feeling) when they are prepared into a dry suspension dosage form; additionally, since micropellet encapsulation is surface encapsulation (that is, a coating solution is sprayed on the surface of API micropellets and dried to form coated micropellets to achieve an encapsulation effect), the API release rate when preparing micropellets into a suspension (pH 6.8) in an in vitro environment is between 0.5-2% or higher, and the taste-masking effect is not good when oral suspensions are prepared for drugs with extremely bitter API. Therefore, the pellet coating technique has a poor taste-masking effect on vonoprazan fumarate.

For the macroporous resin adsorption technique, macroporous resins have an adsorption effect; however, since the resins have a low adsorption rate, generally 5-20%, the proportion of resins is 80% or higher, leading to a high proportion of resin when taking drugs, which is prone to side effects; at the same time, the appearance of the product is not completely spherical, and the fluidity of the product is not good.

The taste modulation technique achieves taste masking by adding auxiliary materials such as a flavoring agent. Adding auxiliary materials reduces the bitter taste by interfering with taste buds. The method is the simplest and low-cost, but the effect is limited, with poor effect on extremely bitter and water-soluble medicaments. Therefore, the taste modulation technique has a poor taste-masking effect on vonoprazan fumarate.

Microspheres refer to spheres with particle sizes at the micron level. Medicament-loaded microspheres in the field of medicament delivery refer to tiny spheres or spheroids formed by dissolving or dispersing a medicament in a polymer material, with a particle size generally in the range of 1-250 µm. The principle of medicament loading of microspheres is to embed or adsorb a medicament on the surface or inside of a polymer by physical means. Microspheres can be classified into porous microspheres, double-layer microspheres, and magnetic microspheres according to their structure. The microsphere technique is applied in the pharmaceutical field, mainly for injections and oral formulations. Currently, microsphere preparation technologies include an emulsification-volatilization method, a phase separation method, a spray drying method, a hot-melt extrusion method, etc.

The basic principle of the emulsification-volatilization method is as follows: organic solvents dissolving a medicament and polymer are added into a continuous phase (aqueous phase) solution containing a surfactant under the condition of high-speed stirring to form an emulsified solution, and the organic solvents in the emulsion are volatilized by physical conditions (vacuum, stirring or film evaporation, etc.); with the rapid diffusion and removal of the solvents with higher solubility or better volatility in the continuous phase, the external polymer of the emulsion droplet precipitates, solidifies and deposits to form a thin film; and with the further diffusion and volatilization of the organic solvents, the internal polymer solidifies and shrinks from the inside to the outside, forming a cavity in the center, thus achieving the solidification of microspheres. The emulsification-volatilization method is typically suitable for preparing microspheres of medicaments with poor water solubility. The residual organic solvents in the product are difficult to remove, and the encapsulation efficiency of microspheres is low, ranging from 20-80%.

The principle of the phase separation method to prepare microspheres is essentially extraction technology. A third component, generally an organic non-solvent, is added to a polymer-medicament-solvent system under stirring. The solubility of the polymer decreases with the addition of the third component, such that the two phases aggregate at a specific point, causing the phase separation of the solvent from the polymer to form very soft medicament-loaded droplets, and the system is then transferred to another organic non-solvent to solidify the microspheres to obtain the final microspheres. The phase separation method is suitable for medicaments with strong water solubility and has complicated operation steps and poor process reproducibility, which is not conducive to industrial production. Moreover, a variety of organic solvents are used, with large amounts and high unit consumption, and the microsphere products are prone to high residual solvents. In addition, the encapsulation efficiency of the microspheres is low, generally around 20-80%.

The principle of the spray drying method to prepare microspheres is to spray liquid raw materials and liquid auxiliary materials into a hot drying medium, transforming the raw materials and auxiliary materials into dry powder in spray drying equipment. The spray drying method is suitable for medicaments with strong heat sensitivity. It requires exclusive drying equipment and has high energy consumption, resulting in microspheres with incomplete appearance and irregular granular surfaces.

The principle of the hot-melt extrusion method to prepare microspheres is as follows: prescribed amounts of a medicament and auxiliary materials are mixed and heated for melting, and then extruded through a sieve plate and quickly cooled to prepare a strip-shaped solid, which is then crushed to make a microsphere product. The hot-melt extrusion method is suitable for medicaments with strong heat resistance. The microsphere products have poor roundness of appearance, and some are still in the shape of columnar particles with poor fluidity. The particle size of the microsphere products is relatively large, ranging from 200-1000 microns.

Therefore, how to develop a microsphere preparation process with a good taste-masking effect, high encapsulation efficiency, wide adaptability (applicable for both water-soluble and lipid-soluble medicaments), small particle size, high product roundness, high fluidity, and low residual solvent content is an urgent problem to be solved.

In addition, Helicobacter pylori (Hp) is a helical, gram-negative, microaerobic bacterium. Helicobacter pylori infection is the most important cause of chronic gastritis, peptic ulcer and gastric mucosa-associated lymphoid tissue lymphoma. It is one of the important causes of gastric cancer, and the population is generally susceptible to Hp. In recent years, the drug resistance of clarithromycin, metronidazole, etc., has increased year by year, and the clinical failure rate of conventional PPI triple and quadruple diagnosis and treatment schemes is constantly increasing. Therefore, the eradication and the resistance of medicaments against Hp infection also need to be solved urgently. Currently, there is no anti-Hp infection compound formulation product based on P-CAB inhibitors (such as vonoprazan fumarate).

### Summary of the Invention

An objective of the present disclosure is to overcome the deficiencies of the prior art and provide a taste-masking microsphere with good taste-masking effects and wide adaptability (applicable for both water-soluble and lipid-soluble medicaments), a preparation process therefor, as well as a composition comprising the taste-masking microspheres of vonoprazan fumarate with significant anti-Hp infection effect.

In the present disclosure, by optimizing the preparation process, the prepared taste-masking microsphere has the advantages of high yield and encapsulation efficiency, small particle size, high product roundness, high fluidity, low residual solvent content, and excellent comprehensive performance.

The present disclosure is achieved by the following technical solutions:
A taste-masking microsphere comprises the following components in parts by weight: 1.5-21.2 parts of a medicament ingredient, 35-110 parts of a medicament carrier, 0-30 parts of an alkalizing agent, and 0-8 parts of a plasticizer.

Preferably, the taste-masking microsphere comprises the following components in parts by weight: 5-10 parts of the medicament ingredient, 50-90 parts of the medicament carrier, 0-8 parts of the alkalizing agent, and 2-8 parts of the plasticizer.

More Preferably, the taste-masking microsphere comprises the following components in parts by weight: 5-10 parts of a medicament ingredient, 50-90 parts of a medicament carrier, 2-8 parts of an alkalizing agent, and 2-8 parts of a plasticizer.

Preferably, the taste-masking microsphere further comprises at least one of an opacifier and an auxiliary agent. The auxiliary agent is used to increase the density and fluidity of the microspheres, improve the stability of the medicament ingredient of microspheres, and the like.

More preferably, the opacifier includes at least one of zinc oxide and titanium dioxide, further preferably titanium dioxide.

More preferably, the auxiliary agent includes at least one of fumaric acid, succinic acid, dibutyl hydroxytoluene, magnesium stearate, silica, talc powder, and povidone; further preferably at least one of fumaric acid, succinic acid, dibutyl hydroxytoluene, silica, and talc powder.

Preferably, the medicament carrier includes a carrier A and a carrier B, wherein the carrier A is a pH-dependent carrier or a pH-independent carrier, and the carrier B is a matrix-type carrier.

The pH-dependent carrier is pH-dependent, which is insoluble in an in vitro aqueous solution (about pH 6.8), thereby achieving a taste-masking effect, and is soluble in vivo (pH 1-4), enabling API to be dissolved to take effect.

The pH-independent carrier is not affected by pH and swells in water to form pores, thereby allowing the dissolution of API to take effect.

More preferably, the carrier A includes at least one of a polyacrylic resin, a methyl methacrylate-diethylaminoethyl methacrylate copolymer, and a polyvinylacetal diethylaminoacetate.

More preferably, the polyacrylic resin includes at least one of Eudragit EPO, Eudragit E100, Eudragit RLPO, Eudragit RL100, Eudragit RSPO, and Eudragit RS100.

More preferably, the methyl methacrylate-diethylaminoethyl methacrylate copolymer is Kollicoat Smartseal 30D100P.

More preferably, the pH-dependent carrier includes at least one of Eudragit EPO and Eudragit E100.

More preferably, the pH-dependent carrier includes Eudragit EPO.

More preferably, the pH-independent carrier includes at least one of Eudragit RLPO, Eudragit RL100, Eudragit RSPO, and Eudragit RS100.

More preferably, the pH-independent carrier includes at least one of Eudragit RLPO, and Eudragit RSPO.

More preferably, the pH-independent carrier includes Eudragit RLPO and Eudragit RSPO, in a mass ratio of 1 : 1 - 8 : 1.

More preferably, the amount of the carrier A is 5-40 parts, preferably 10-30 parts.

More preferably, the carrier B (matrix-type carrier) includes at least one of ethylcellulose, microcrystalline cellulose, sodium carboxymethyl cellulose, cellulose acetate, polyvinyl acetate, ammonio methacrylate copolymer type A, ammonio methacrylate copolymer type B, crospovidone, and maltodextrin.

More preferably, the ethylcellulose includes at least one of N7, N10, N22, and N50. N denotes ethylcellulose, and the following number denotes the type number.

More preferably, the matrix-type carrier includes at least one of N7 and N10.

More preferably, the amount of the matrix-type carrier is 30-70 parts, and more specifically may be 30-60 parts, or 40-60 parts.

Preferably, the alkalizing agent includes at least one of sodium carbonate, sodium bicarbonate, magnesium oxide, meglumine, and tromethamine.

The alkalizing agent can improve the solubility of the API (i.e., active pharmaceutical ingredient) raw material, and after adding the alkalizing agent, the reaction stirring time is controlled to be 0-12 h, preferably 0.66-2 h.

More preferably, the alkalizing agent comprises at least one of sodium bicarbonate, and magnesium oxide.

The plasticizer can reduce the aggregation and adhesion of the microspheres in the sphere formation and solidifying processes.

Preferably, the plasticizer includes at least one of diethyl phthalate, tributyl citrate, polyethylene glycol 6000, and triethyl citrate.

More preferably, the plasticizer is triethyl citrate.

Preferably, the medicament ingredient is a compound with bitter taste or off flavor, further preferably an alkaloid compound.

More preferably, the medicament ingredient is at least one of an organic amine alkaloid and a nitrogen-containing heterocyclic alkaloid.

The formulation of the microspheres of the present disclosure has an excellent taste-masking effect on API with intense bitter taste, bitter taste, and specific off flavor. Generally, bitter APIs are alkaloid compounds, such as organic amine alkaloids, nitrogen-containing heterocyclic alkaloids, etc. Alkaloid molecules contain nitrogen atoms, and the outermost electrons of the nitrogen atoms can combine with protons (H') in acids by coordinate covalent bonds to form salts. Free alkaloids are generally insoluble or poorly soluble in water, and after salt formation, their water solubility and stability are enhanced. Most alkaloids have a bitter or pungent taste, and their bitterness is enhanced after salt formation.

More preferably, the medicament ingredient includes at least one of vonoprazan, a pharmaceutically acceptable salt of vonoprazan, berberine hydrochloride, sildenafil citrate, azithromycin, cefuroxime axetil, vardenafil hydrochloride, metformin hydrochloride, paroxetine hydrochloride, sitagliptin hydrochloride, sertraline hydrochloride, pyridostigmine bromide, lidocaine hydrochloride, famotidine, ibuprofen, tramadol hydrochloride, allitride, bepotastine besilate, acetaminophen, colchicine, racecadotril, fluoxetine hydrochloride, flucloxacillin sodium, lacosamide, clarithromycin, donepezil hydrochloride, rivaroxaban, linezolid, cefcapene pivoxil, cefetamet pivoxil, meropenem, moxifloxacin, fluvoxamine maleate, rebamipide, cefteram pivoxil, verapamil, quetiapine, amisulpride, sulpiride, metoprolol, pravastatin, atomoxetine, escitalopram, tilidine hydrochloride, ondansetron, vortioxetine hydrobromide, domperidone, zopiclone, roxatidine, loperamide, diphenhydramine, epinastine, mirabegron, solifenacin, irbesartan, vortioxetine hydrobromide, ticagrelor, captopril, colesevelam hydrochloride, rasagiline, nintedanib esylate, enalapril maleate, apremilast, piroxicam, flutamide, varenicline tartrate, pazopanib, pramipexole, ripretinib, tamsulosin hydrochloride, risperidone, linagliptin, teneligliptin, dabigatran etexilate, odevixibat, micafungin, aprepitant, roflumilast, cefprozil, cefpodoxime proxetil, and tenapanor hydrochloride.

More preferably, the pharmaceutically acceptable salt of vonoprazan is at least one of a fumarate, L-malate, succinate, hemi-L tartrate, dihydrogenphosphate, disulfate, sulfate, hydrochloride, mesylate, phosphate, acetate, citrate, maleate, tartrate, bitartrate, and hydrobromide of vonoprazan. It may have good effects in taste masking, solubility and the like based on all the above salts.

Preferably, the mass of the medicament ingredient accounts for 1.5-21.2%, more specifically may be 5-15%, and 5-10% of the total mass of the components of the taste-masking microsphere.

Vonoprazan is an organic amine compound, which is alkaline and can form a fumarate salt. Vonoprazan fumarate has an intense bitter taste. By utilizing the microsphere taste masking technology of the present disclosure, the intense bitter taste of vonoprazan fumarate can be reduced to basically no bitterness, which is beneficial to the development and application of oral solution (suspension) formulations, meeting the medication requirements of children, the elderly, and patients with dysphagia.

Preferably, when the medicament ingredient is vonoprazan fumarate, the auxiliary agent includes at least one of fumaric acid, succinic acid, and dibutyl hydroxytoluene, wherein the addition amount of the fumaric acid accounts for 0.08-12.42% of the total mass of the components of the taste-masking microsphere, the addition amount of the succinic acid accounts for 0.08-2% of the total mass of the components of the taste-masking microsphere, and the addition amount of the dibutyl hydroxytoluene accounts for 0.08-1% of the total mass of the components of the taste-masking microsphere. Fumaric acid, succinic acid, or dibutyl hydroxytoluene can improve the stability of products.

More preferably, the addition amount of the fumaric acid accounts for 0.08-10%, more specifically may be 0.08-5% of the total mass of the components of the taste-masking microsphere.

The present disclosure further relates to a preparation process for taste-masking microspheres, including the following steps:
(1) preparing a dispersed phase of microspheres: dissolving the components of the taste-masking microspheres in a solvent A as a dispersed phase of microspheres;
(2) preparing a continuous phase for microspheres: selecting a solvent B as the continuous phase for microspheres; and
(3) pouring the continuous phase for microspheres into a reactor, then feeding the dispersed phase of microspheres thereto from the bottom of the reactor, forming spheres by dispersion, followed by solidifying, filtering, washing, and drying of the spheres.

In the preparation process of the taste-masking microsphere of the present disclosure, the dispersed phase and the continuous phase are mixed and sequentially undergo the stages of formation of spherical droplets (referred to as droplets), formation of soft spheres, solidification of sphere walls, solidification of spheres, filtration of microspheres, and drying of microspheres.

Preferably, the microspheres are washed after microsphere filtration and then dried to obtain the taste-masking microspheres.

Spherical droplet formation stage:
The dispersed phase of microspheres is fed at the bottom of the continuous phase for microspheres to gradually form spherical droplets.

With the adjustment and control of the spheronization and dispersibility of the materials, undispersed material units (i.e., large spheres) are prevented from running to the liquid surface and bursting to form films; and the materials are prevented from being too dispersible to form spherical droplets, forming emulsion.

Soft sphere formation stage:
With the rapid diffusion of the solvent on the surface of the spherical droplets into the continuous phase, the medicament carrier on the surface of the spherical droplets precipitates to form a thin and soft pellicle, forming soft spheres.

Sphere wall solidifying stage:
1) With the diffusion of the solvent in the soft sphere into the continuous phase for microspheres and the reverse diffusion of the solvent in the continuous phase for microspheres into the soft sphere, the wall of the soft sphere gradually solidifies, and the medicament carrier pellicle on the surface of the soft sphere gradually thickens, forming a cored sphere (the core of the sphere is still liquid) with a shell having a certain thickness.
2) The solvent in the cored sphere further diffuses over time, and the thickness of the shell of the cored sphere is increasing.

Sphere solidifying stage:
After the cored spheres are completely solidified, they become solid spheres or hollow spheres with a small particle size. The surface of the microspheres has a high density, while the interior is porous, and the medicament ingredients are inside the microspheres in the form of molecular dispersions, which is more conducive to the delivery and release of the medicament.

Microsphere washing stage:
The solvents, surfactants, etc., on the surface of the microspheres are washed off. It is preferred to wash with purified water at a pH of 6.5-7.5.

Microsphere drying stage:
Drying in the presence of temperature gradients is preferred, and it is further preferred to carry out air-blast drying at room temperature for 1 hour and then drying for 2 hours after heating to 40°C.

The oral microspheres of the present disclosure mainly have a diameter of 50-380 microns (which is larger than that of the injectable microspheres), and after the initial formation of the microspheres, a certain period of time is required to enable the solvent to diffuse in the continuous phase for microsphere to solidify the microspheres. The adhesion and aggregation of microspheres in the continuous phase and the bursting of microspheres to form a film when the microspheres reach the liquid level before the shell is solidified will lead to the failure of sphere formation. These two phenomena are the priority problems to be solved by the preparation process of microspheres, and how to inject the materials is an important means to solve the problems.

Feeding is carried out below the liquid surface (conventionally above the liquid surface), and the dispersion effect during the sphere formation by injection is adjusted and controlled through the feeding position of the reactor to control the particle size and particle size uniformity of spheres. The materials may be fed from the bottom, middle part, and upper part of the liquid continuous phase for the microspheres in the reactor, preferably from the bottom.

Preferably, the solvent A in step (1) is a mixed solvent of an organic solvent C and water.

More preferably, the mass percentage of the water in the solvent A is 0-35%, preferably in a range of 0-33.5%, further preferably 0-10.5%, or optionally in a range of 3.5-33.5%, and more specifically may be 7.5-10.5%.

Water can increase the solubility of a water-soluble API, enabling the adjustment and control of the diffusion speed of the dispersed phase of microspheres in the continuous phase for microspheres, and thus the surface porosity of microspheres and the hollow ratio inside microspheres.

More preferably, the organic solvent C includes at least one of methanol, ethanol, isopropanol, 1,2-propanediol, acetone, tetrahydrofuran, methyltetrahydrofuran, toluene, xylene, acetonitrile, N,N-dimethylacetamide, ethyl acetate, n-butanol, dichloromethane, trichloromethane, tetrachloroethane, and methylpropylene glycol acetate.

When preparing the dispersed phase of microspheres, the selection of solvent C should consider the following three factors, including:
1) Selecting a solvent enabling the sphere formation in the continuous phase for microspheres: including at least one of ethyl acetate, n-butanol, dichloromethane, trichloromethane, tetrachloroethane, and methylpropylene glycol acetate; based on comprehensive evaluations from aspects such as spheronization, circular economy, the solubility of medicament carrier, and use safety, preferably at least one of ethyl acetate, n-butanol and dichloromethane, further preferably at least one of ethyl acetate and n-butanol.
2) Selecting a solvent with good solubility to the medicament carrier (provided that the solvent has spheronization in the continuous phase for microspheres): including at least one of ethyl acetate, dichloromethane, n-butanol and methylpropylene glycol acetate; preferably at least one of ethyl acetate and n-butanol.
3) Selecting a solvent with good solubility to API and good dispersibility in the continuous phase for microspheres: including at least one of methanol, ethanol, isopropanol, 1,2-propanediol, acetone, tetrahydrofuran, methyltetrahydrofuran, toluene, xylene, acetonitrile, and N,N-dimethylacetamide, preferably at least one of methanol, ethanol, isopropanol and acetone.

More preferably, the solvent C in step (1) includes at least one of methanol, ethanol, isopropanol, acetone, ethyl acetate, n-butanol, and dichloromethane.

In another example of the present disclosure, the solvent C is a mixed solvent of two solvents, specifically including any one of the following mixed solvents:
a) an ethyl acetate/ethanol mixed solvent, wherein when the medicament carrier comprises a pH-dependent carrier, the volume ratio thereof is 4 : 1 - 1 : 1, preferably 1.5 : 1; and when the medicament carrier comprises a pH-independent carrier, the volume ratio thereof is 25 : 1 - 5 : 1, preferably 20 : 1;
b) a n-butanol/ethanol mixed solvent with a volume ratio of 3 : 1 - 1.2 : 1, preferably 1.5 : 1;
c) a dichloromethane/ethanol mixed solvent with a volume ratio of 1.7 : 1- 1 : 1, preferably 1.5 : 1;
d) an ethyl acetate/methanol mixed solvent with a volume ratio of 3 : 1 - 1.15:1, preferably 1.5 : 1;
e) an ethyl acetate/isopropanol mixed solvent with a volume ratio of 4 : 1 - 1.2 : 1, preferably 1.5 : 1;
f) an ethyl acetate/acetone mixed solvent with a volume ratio of 2 : 1 - 1 : 1.2, preferably 1.5 : 1; and
g) an ethyl acetate/acetonitrile mixed solvent with a volume ratio of 2 : 1 - 1 : 1, preferably 1.5 : 1.

In another example of the present disclosure, the solvent C is a mixed solvent of three solvents, specifically including any one of the following mixed solvents:
(a) a mixed solvent of ethyl acetate, ethanol, and n-butanol with a volume percentage selected from the following ranges:
   60% of ethyl acetate, 30-10% of ethanol, and 10-30% of n-butanol; preferably 60% of ethyl acetate, 20% of ethanol, and 20% of n-butanol;
(b) a mixed solvent of ethyl acetate, ethanol, and methanol with a volume percentage selected from the following ranges:
   60% of ethyl acetate, 30-10% of ethanol, and 10-30% of methanol; preferably 60% of ethyl acetate, 20% of ethanol, and 20% of methanol;
(c) a mixed solvent of ethyl acetate, ethanol, and isopropanol with a volume percentage selected from the following ranges:
   60% of ethyl acetate, 30-10% of ethanol, and 10-30% of isopropanol; preferably 60% of ethyl acetate, 20% of ethanol, and 20% of isopropanol; and
(d) a mixed solvent of ethyl acetate, ethanol, and acetone with a volume percentage selected from the following ranges:
   60% of ethyl acetate, 30-10% of ethanol, and 10-30% of acetone; preferably 60% of ethyl acetate, 20% of ethanol, and 20% of acetone.

Preferably, the solvent B in step (2) includes water or a mixed solvent of at least one of ethyl acetate, ethanol, liquid paraffin and dichloromethane with water.

More preferably, the solvent B in step (2) is water.

Preferably, the continuous phase for microspheres in step (2) further includes a surfactant for improving the surface tension of the continuous phase.

More preferably, the amount of the surfactant in the continuous phase for microspheres is 0.01-2 wt%, preferably 0.05 wt% - 0.8 wt%.

More preferably, the surfactant includes at least one of sodium oleate, a Tween series surfactant, polyvinyl alcohol, sodium dodecyl sulfate, and sodium carboxymethyl cellulose.

More preferably, the Tween series surfactant includes at least one of Tween 20, Tween 40, Tween 60 and Tween 80, preferably Tween 80.

Regarding the preferred surfactants:
1) The mass percentage of sodium oleate in the continuous phase for microspheres is in a range of 0.01-0.05%.
2) The mass percentage of Tween 80 in the continuous phase for microspheres is in a range of 0.02-2%.
3) The polyvinyl alcohol surfactants include types 03-88, 05-88, 17-88, 20-88, 25-88, etc., preferably 05-88; and the concentration of the polyvinyl alcohol aqueous phase is in a range of 0.05-2.0%, preferably 0.2-0.4%.
4) The mass percentage of sodium dodecyl sulfate (SDS) surfactants in the continuous phase for microspheres is controlled in a range of 0.02-0.2%, preferably 0.05%.
5) The mass percentage of sodium carboxymethyl cellulose (CMC-Na) surfactants in the continuous phase for microspheres is controlled in a range of 0.02-0.25%, preferably 0.05%.

Preferably, the continuous phase for microspheres in step (2) has a temperature of 2-30°C.

Under such a temperature condition, the quality stability of the API is high during the formation of spheres, with a high microsphere yield.

More preferably, the continuous phase for microspheres in step (2) has a temperature of 2-10°C.

Preferably, the continuous phase for microspheres in step (2) has a pH of 6-11.

Under such a pH condition, the diffusion of the dispersed phase in the continuous phase and the quality of the formed spheres are more stable.

Preferably, the continuous phase for microspheres in step (2) has a pH of 6-8, more specifically may be 7-8.

Preferably, the mass ratio of the continuous phase for microspheres to the dispersed phase of microspheres is 14.7-123.5 : 1.

More preferably, the mass ratio of the continuous phase for microspheres to the dispersed phase of microspheres is 20-50 : 1.

Preferably, the ratio of the total mass of the components of the taste-masking microsphere to the mass of solvent A is: 6.5-21.5 : 100.

More preferably, the ratio of the total mass of the components of the taste-masking microsphere to the mass of solvent A is 6.5-17 : 100, and more specifically may be 6.5-15 : 100, 12-17 : 100, and 12-15 : 100.

Preferably, the reactor in step (3) is in the shape of an inverted triangular pyramid frustum, with a feed port provided at the bottom of the reactor.

More preferably, the cross section of the inverted triangular pyramid frustum is an isosceles triangle.

More preferably, the isosceles triangle has a base angle of β, and the value of β is in a range of 45° - 75°, preferably 60°.

More preferably, the height of the reactor is 10 cm - 200 cm.

More preferably, the included angle between a side plane edge and the bottom face of the inverted triangular pyramid frustum is α, and the value of α is in a range of 30° ≤ α < 90°, preferably 60° ≤ α < 90°, further preferably α = 79° - 85°.

Preferably, 2-4 layers of stirring blades are provided in the reactor, with a stirring rotational speed of 50-900 rpm.

More preferably, the blade is a push-down stirring paddle.

Preferably, a washing operation is carried out after filtration in step (3).

In the present disclosure, by developing a novel microsphere reactor and a microsphere process, the spiral rising path and rising speed of the formed spherical droplets are controlled, and the rising time of the spherical droplets in the aqueous phase is reasonably controlled, thus preventing the spherical droplets from reaching the liquid surface before becoming soft spheres to burst at the air/water interface to form an organic film.

The principle of preparing taste-masking microspheres by the bottom diffusion method of the present disclosure is as follows:
The medicament ingredient and the medicament carrier are dissolved into a solvent, and the spheronization and dispersibility of the dispersed phase of medicament-containing microspheres in the continuous phase for microspheres are adjusted fully utilizing the lipid-aqueous solubility partition coefficient of the solvent; the dispersed phase of the medicament-containing microspheres is dispersed at the bottom of the continuous phase for microspheres and forms uniform spheres; and due to the density difference between the dispersed phase of microspheres and the continuous phase for microspheres, the formed spherical droplets move towards the liquid surface while the solvent is diffused and solidified, and solidify into semi-solidified (sphere-wall solidified) microspheres with certain hardness and mechanical resistance before reaching the liquid surface.

Further, with the use of the reactor in the shape of an inverted triangular pyramid frustum and multi-layer downward stirring, the fluid has the effects of horizontal switchback (as shown in FIG. 1) and longitudinal vortex (as shown by mark A in FIG. 2 and FIG. 3) at the same time, so that the microspheres start to rise in a curve from the bottom of the reactor, and the rising trajectory is shown in FIG. 4. The microspheres are prevented from reaching the liquid surface and bursting to form a film by controlling the solidification and rising time of the microspheres, and the aggregation and adhesion of the microspheres are avoided by the switchback effect in the horizontal direction of the inverted triangular pyramid frustum reactor, thereby achieving the good effect of uniform particle size of microspheres.

To control the stable and continuous diffusion of the dispersed phase of microspheres in the continuous phase for microspheres and to control inter-batch and intra-batch differences, a prescribed amount of solvent for the dispersed phase of microspheres is added to the initial continuous phase for microspheres, and the continuous phase mother liquid is reused during batch feeding or continuous feeding.

Preferably, the filtrate obtained after the filtration in step (3) (i.e., the continuous phase mother liquid) is reused in the next reaction, with a reuse proportion (i.e., the percentage by mass of the filtrate relative to the continuous phase for microspheres added next time) of 10-80%, preferably 30-50%; alternatively, the solvents in the filtrate obtained after the filtration in step (3) are recovered first, and then the recovered solvents are used for the preparation of the solvent A or solvent B.

If the solvents recovered in step (3) contain ethanol and are used for preparing solvent A, the application quality standard for ethanol is a mass concentration of 42.3% - 99.9%, further preferably 75% - 80%.

The circular reuse of the continuous phase mother liquid can control the solvent diffusion rate during the initial formation of microspheres, improve encapsulation efficiency, and reduce the recovery and emission of organic solvents.

Preferably, before reusing the continuous phase mother liquid, the organic solvents in the continuous phase mother liquid are first recovered using organic membrane recovery technology, a vaporized membrane is formed on the surface of the organic membrane using a vacuum system, and the vaporized organic solvents pass through the organic membrane under the action of the pressure difference on both sides of the film, followed by condensation and cooling to obtain a mixed organic solvent liquid with a content of 90% or higher. After content detection and secondary blending, the recovered mixed solvent can be directly used in production, such that the investment in equipment such as large-scale solvent recovery column, and the energy consumption are reduced, achieving a circular economy and green economy.

Preferably, the preparation process is a continuous process, in which a continuous phase for microspheres (which is prepared by mixing a new continuous phase for microspheres and the continuous phase mother liquid in proportions) is continuously supplemented at the bottom of the reactor through a feed pump, a suspension containing microspheres is continuously discharged through a liquid level controller from the middle part of the microsphere reactor, then the suspension is filtered and washed to obtain wet microspheres, and the filtered mother liquid is recovered via an organic membrane and reused.

Therefore, the microsphere preparation process of the present disclosure is green, environmentally friendly, has a circular economy, and can realize continuous production.

The present disclosure further relates to a composition, comprising the taste-masking microspheres containing a vonoprazan fumarate ingredient described above, or the vonoprazan fumarate-containing taste-masking microspheres obtained by the preparation process described above.

Preferably, the composition comprises, in parts by weight, 10-20 parts of the taste-masking microspheres, 500-1500 parts of amoxicillin, and 50-150 parts of rifabutin.

More preferably, the mass ratio of the taste-masking microspheres, amoxicillin, and rifabutin is 20 : 1000 : 50, 20 : 1000 : 75, 20 : 1000 : 100, 20 : 1000 : 125, or 20 : 1000 : 150.

More preferably, the amoxicillin and/or rifabutin is in the form of a granule, a micropellet, or a microsphere.

The present disclosure further relates to the use of the composition described above in the preparation of a medicament against Helicobacter pylori infection related diseases.

The present disclosure further relates to a formulation, comprising the taste-masking microspheres described above, or the taste-masking microspheres obtained by the preparation process described above, or the composition described above.

Preferably, the dosage form of the formulation is a dry suspension, a granule, a capsule, a tablet, or a film.

The beneficial effects of the present disclosure are as follows:
Good taste-masking effect: the taste-masking microspheres of vonoprazan fumarate of the present disclosure, when prepared into a suspension (pH 6.8) in an in vitro environment, has an API release rate of less than 0.5%, a reduction rate of bitterness/off-flavor level in a taste test of greater than 90%, a reduction rate of base salt-like bitterness in an electronic tongue test of greater than 85%, and a reduction rate of alkaline bitterness reaching 100%, with an extremely excellent taste-masking effect, making it possible to develop a single and compound formulation of medicament ingredients in the dosage form of a dry suspension, and providing new formulations with high compliance and convenient administration for children, the elderly and patients with dysphagia.

Microspheres with small particle size, resulting in oral suspension having no gritty feeling: in the present disclosure, the particle size of the taste-masking microspheres is mainly controlled to be 50-380 microns, and the product has a small particle size, concentrated particle size distribution, and no gritty feeling; however, the products prepared by a conventional granulation technique or micropellet coating technique have a particle size of 300-1200 microns and a noticeable gritty feeling. The present disclosure solves the problem of the gritty feeling in oral administration of a dry suspension preparation, improves patient comfort and compliance during medication, and is more suitable for children, and elderly patients with dysphagia.

Good taste-masking effects on various alkaloid medicaments with intense bitterness or off flavor: The present disclosure has a good taste-masking effects on compounds with bitter taste or off flavor, an in vitro release rate of 0.5% or less, a reduction rate of bitterness/off-flavor level in a taste test of greater than 85%, no gritty feeling, and a significant taste effect, thus improving medication compliance.

High microsphere product yield and encapsulation efficiency: In the present disclosure, the microspheres are prepared using a bottom diffusion method, and a dispersed phase with solubility, spheronization, and dispersibility is obtained by optimizing the composition of the dispersed phase and continuous phase. Generally, the microsphere preparation processes have a yield and an encapsulation efficiency of 60% or higher, and an encapsulation efficiency generally between 60% - 80% under normal temperature conditions. After the formulation process is optimized, the encapsulation efficiency may reach 90% or higher, and the encapsulation efficiency of microspheres is up to 94.6%.

A microsphere process realizing continuous and automated production: the preparation process of the present disclosure can achieve an excellent encapsulation effect, taste-masking effect, and the like when used for both water-soluble and lipid-soluble medicaments. The present process is simple, requires no complicated equipment and easily available raw materials, is suitable for continuous, automated, and large-scale production, and is widely applicable. The taste-masking microspheres prepared by the present disclosure have high integrity and roundness, smooth and dense external surfaces, uniformly dispersed medicament ingredients in the microspheres in the form of molecular dispersion, good encapsulation effect, and fast dissolution speed of the medicament in the stomach.

By innovatively designing the reactor in the shape of an inverted triangular pyramid frustum and using a push-down stirring paddle, the present disclosure achieves transverse switchback and longitudinal vortex effects of fluid mechanics, solving the problems of adhesion (aggregation) and bursting to form a film of microspheres during the sphere formation and solidification processes.

The present disclosure solves problems such as API compatibility and mixing uniformity in compound formulations of triple compound dry suspension. Utilizing the fact that various APIs exist in the mixed powder in the form of individual microspheres or particles, the compatibility of APIs with auxiliary materials during storage is overcome.

The composition of the present disclosure includes taste-masking microspheres containing a vonoprazan fumarate ingredient, amoxicillin and rifabutin, which can realize clinical effectiveness and safety when used for treatment against Hp (eradication of Helicobacter pylori) infection, thus solving the difficult problem of the resistance of clinical medicaments against Hp infection.

The composition of the present disclosure has a fixed composition, which can replace the clinically experiential therapy and greatly simplify the administration procedures. It can be taken after mixing it with water, twice a day, without dietary restrictions, and can improve patient compliance.

### Brief Description of the Drawings

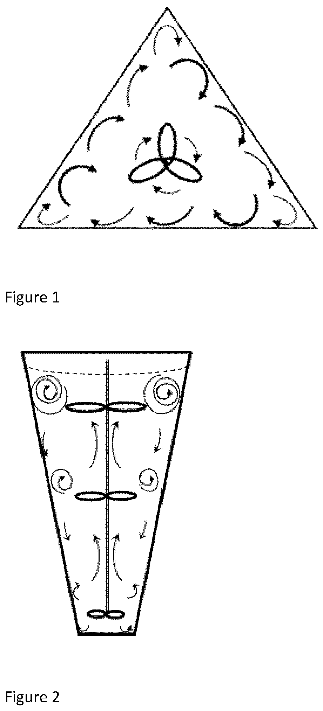

FIG. 1 shows a schematic diagram of the transverse switchback effect of a reactor having a cross section as an isosceles triangle.
FIG. 2 shows a schematic diagram of the longitudinal vortex effect of an inverted triangular pyramid frustum with a push-down stirring paddle.

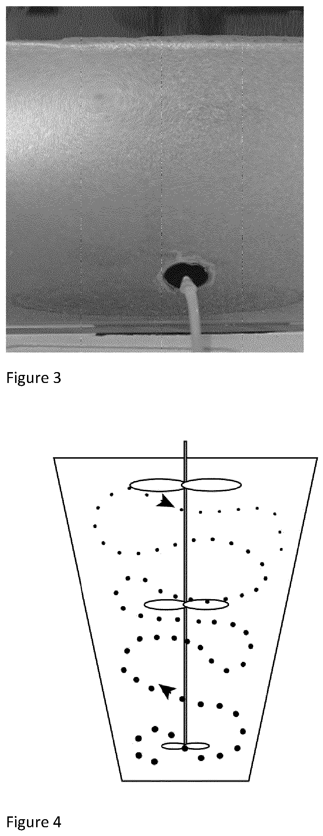

FIG. 3 shows a picture of the longitudinal vortex effect during the preparation of microspheres taken from a side.
FIG. 4 shows a schematic diagram of the rising trajectory of microspheres.

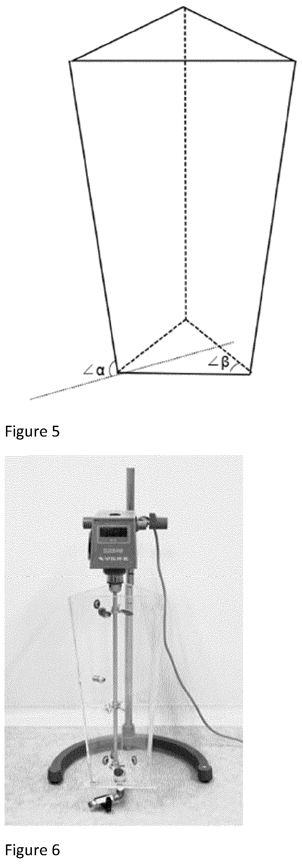

FIG. 5 shows a structural schematic diagram of an inverted triangular pyramid frustum reactor of the present disclosure.
FIG. 6 shows a picture of 1 # reactor.

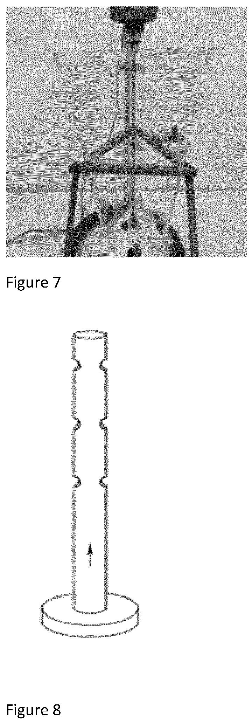

FIG. 7 shows a picture of 2 # reactor.
FIG. 8 shows a structural schematic diagram of a syringe with upper and lower bores.

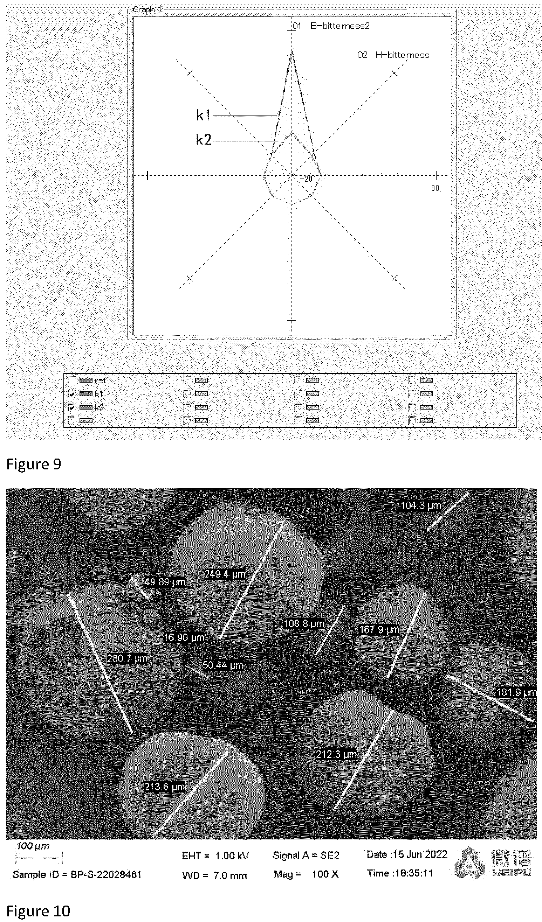

FIG. 9 shows a radar chart of an electronic tongue test of taste-masking microspheres of batch No. FC509-2.
FIG. 10 shows a scanning electron microscope image of the appearance of microspheres of vonoprazan fumarate.

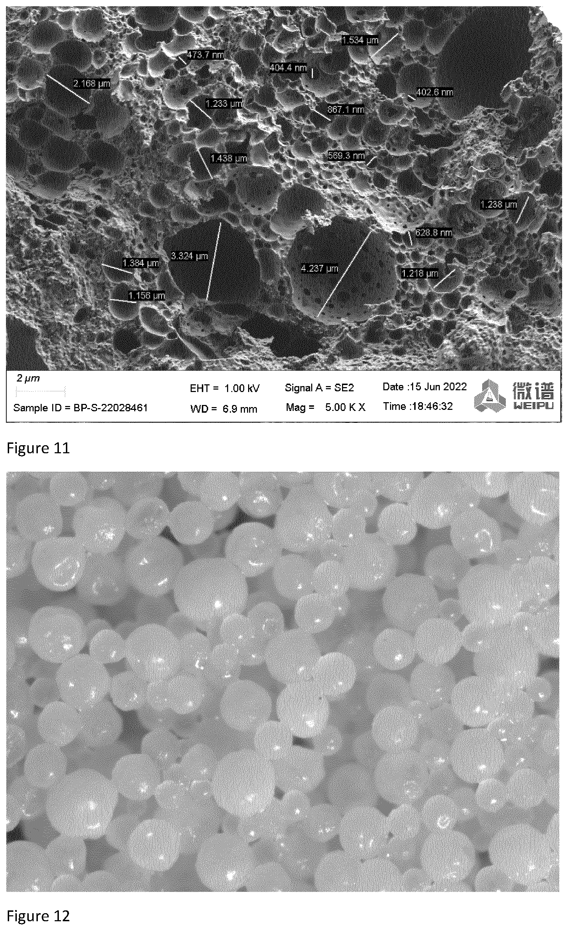

FIG. 11 shows a scanning electron microscope image of the section of microspheres of vonoprazan fumarate.
FIG. 12 shows a microscope image of microspheres of vonoprazan fumarate.

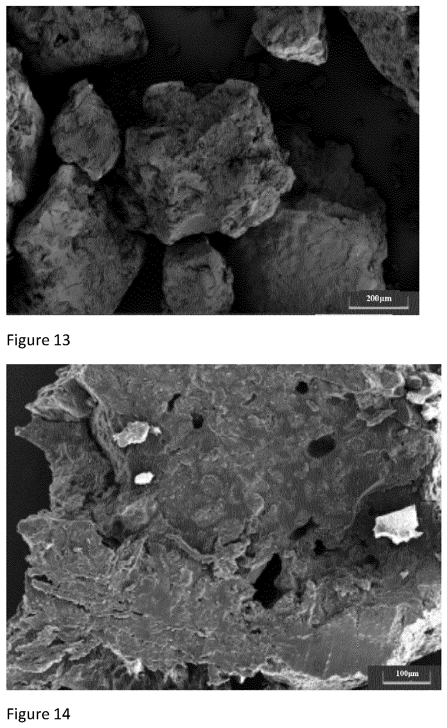

FIG. 13 shows a scanning electron microscope image of the appearance of extruded rounded granules of Comparative example 4.
FIG. 14 shows a scanning electron microscope image of the section of extruded rounded granules of Comparative example 4.

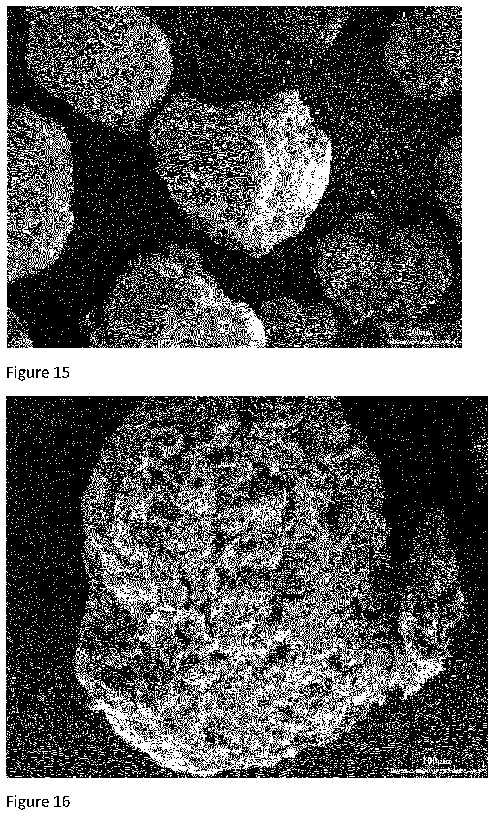

FIG. 15 shows a scanning electron microscope image of the appearance of wet-granulated fluidized-coated granules of Comparative example 5.
FIG. 16 shows a scanning electron microscope image of the section of wet-granulated fluidized-coated granules of Comparative example 5.

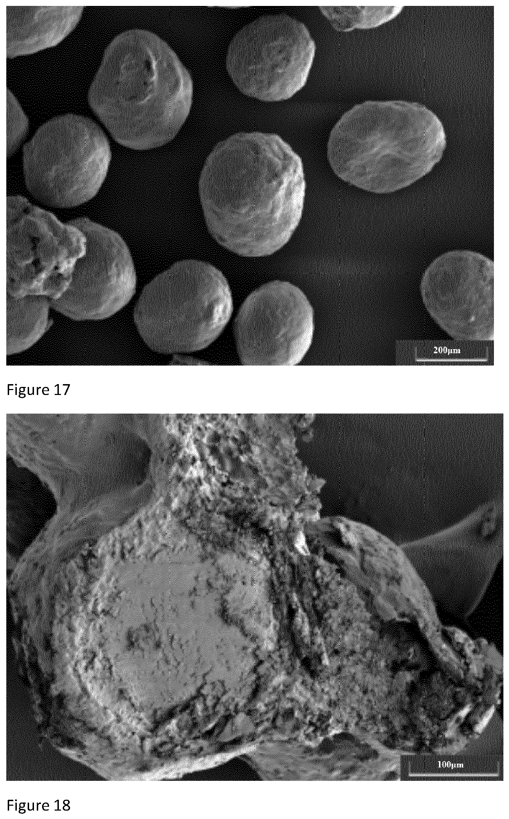

FIG. 17 shows a scanning electron microscope image of the appearance of spray-loaded fluidized-coated micropellets of Comparative example 6.
FIG. 18 shows a scanning electron microscope image of the section of spray-loaded fluidized-coated micropellets of Comparative example 6.

### Detailed Description of Embodiments

The present disclosure is further described below with reference to specific examples, and the advantages and features of the present disclosure will become more apparent as described. However, these examples are merely exemplary and do not limit the scope of the present disclosure in any way. Those skilled in the art should understand that the details and forms of the technical solutions of the present disclosure can be modified or replaced without departing from the spirit and scope of the present disclosure, but these modifications and replacements all fall within the scope of protection of the present disclosure.

The structural schematic diagram of an inverted triangular pyramid frustum reactor of the present disclosure is shown in FIG. 5. The included angle between a side plane edge and the bottom face is α, and the bottom face as an isosceles triangle has a base angle of β.

1# reactor of the present disclosure -- a 3 L transparent triangular pyramid frustum reactor, ∠a = 84.2°, ∠β = 60°, volume: 3 L, equipped with a three-layer push-down stirring paddle, specifically as shown in FIG. 6.

2# reactor of the present disclosure -- an 8 L transparent triangular pyramid frustum reactor, ∠a = 79.1°, ∠β = 60°, volume: 8 L, equipped with a three-layer push-down stirring paddle, specifically as shown in FIG. 7.

In the following examples, API is one of the drug substances, such as vonoprazan fumarate, and the drug substances in the following examples are all vonoprazan fumarate, except for Example 36.

In the following examples, "N7, N10, N22, and N50" are ethylcellulose types, which are sourced from Shenzhen Youpuhui Pharmaceutical Co. Ltd.

In the following examples, the plasticizer is triethyl citrate.

In the following examples, EPO is Eudragit EPO (polyacrylic resin IV powder), E100 is Eudragit E100 (polyacrylic resin IV particles), RSPO is Eudragit RSPO (ammonio methacrylate copolymer Type B powder), and RLPO is Eudragit RLPO (ammonio methacrylate copolymer Type A powder).

In the following examples, 30D100P is Kollicoat Smartseal 30D100P (a methyl methacrylate-diethylaminoethyl methacrylate copolymer).

In the following examples, AEA is polyvinylacetal diethylaminoacetate from Sankyo.

In the following examples, BHT is dibutyl hydroxytoluene, which is sourced from Jiangxi Alpha Hi-tech Pharmaceutical Co., Ltd.

In the following examples, ethanol is absolute ethanol. If aqueous ethanol is used, the amount of water added to solvent A in step (1) can be calculated according to the amount of aqueous ethanol.

### Basic example

Preparation process: The dispersed phase was fed from the bottom of the reactor by means of syringe injection, with 1-2 feed ports on each side, and 3-6 feed ports in total.

The feeding was carried out under stirring at 100-180 rpm for 10-20 min (peristaltic pump model: Signal 2S, feeding rotational speed: 2-15 rpm). After feeding, the stirring speed was 80-120 rpm, and the stirring time was 10-60 min, followed by filtration, washing, and drying to obtain the taste-masking microspheres.

The washing process is specifically: washing twice with purified water at a pH adjusted to 6.5-7.5, 50 mL each time.

The drying process is specifically: air-blast drying at room temperature for 1 hour, and drying for 2 hours after heating to 40°C.

The taste-masking microspheres in each of the following examples were prepared based on this basic example.

### Example 1

Experimental objective: feasibility confirmation and screening of different types of Tween (0.2%) aqueous solutions.

Process conditions: the reactor model was -1#, and the volume of the continuous phase was 3 L.

Solvents of the dispersed phase: ethyl acetate, ethanol (absolute), and water, in a mass ratio of 5.4 : 3.2 : 1 (62 g in total).

Solutes of the dispersed phase: API, EPO, N10, magnesium oxide, triethyl citrate, magnesium stearate, silica, titanium dioxide, and talc powder, in a mass ratio of 5.6 : 15 : 37 : 15 : 5 : 2 : 2 : 1 : 1 (8.36 g in total).

Continuous phase: a series of 0.2% Tween aqueous solutions, at a temperature of normal temperature, and a pH of 7.

Preparation process: with reference to the basic example, the number of the feed ports was 1 on each side, 3 in total; the feeding was carried out under stirring at 150 rpm for 15 min (peristaltic pump model: Signal 2S, feeding rotational speed: 10 rpm); and after feeding, the stirring speed was 100 rpm, and the stirring time was 30 min.

The indexes of the prepared taste-masking microspheres are shown in Table 1.

**Table 1**

| Test batch No. | Tween | Yield (%) | Content^{a} (%) | Encapsulation efficiency (%) | Dissolution rate ^{b} after 30 min (%) |
|---|---|---|---|---|---|
| FC505-5 | 20 | 80.5 | 5.2 | 62.7 | 98.1 |
| FC505-6 | 60 | 86.9 | 4.9 | 63.2 | 102.6 |
| FC325-1 | 80 | 74.0 | 6.3 | 69.2 | 100.3 |

Remarks: the "content" represented by a is the content of API in the final prepared taste-masking microspheres, which also applies to the following examples.

b denotes a 30 min dissolution rate test method: using 900 mL of a pH 4.5 acetate buffer as a dissolution medium, and using a paddle method at 50 rpm at 37°C. The test method applies to the following examples.

### Example 2

Experimental objective: feasibility scope determination and screening and optimization of Tween 80 aqueous solutions with different concentrations.

Process conditions: the reactor model was -1#, and the volume of the continuous phase was 3 L.

Solvents of the dispersed phase: ethyl acetate, ethanol (absolute), and water, in a mass ratio of 5.4 : 3.2 : 1 (62 g in total).

Solutes of the dispersed phase: API, EPO, N10, magnesium oxide, triethyl citrate, and titanium dioxide, in a mass ratio of 5.6 : 15 : 37 : 15 : 5 : 1 (7.86 g in total).

Continuous phase: Tween 80 aqueous solutions, continuous phase temperature: normal temperature, and continuous phase pH: 7.

Preparation process: the same as Example 1.

The indexes of the prepared taste-masking microspheres are shown in Table 2.

**Table 2**

| Test batch No. | Content of Tween 80 (%) | Yield (%) | Content (%) | Encapsulation efficiency (%) | Dissolution rate after 30 min (%) |
|---|---|---|---|---|---|
| FC422-9 | 0.02 | 82.2 | 6.1 | 72.4 | 98.1 |
| FC422-10 | 0.5 | 84.1 | 5.0 | 60.8 | 102.8 |
| FC424-1 | 1 | 87.5 | 5.7 | 68.8 | 100.5 |
| FC424-4 | 2 | 86.9 | 5.4 | 65.5 | 82.0 |

### Example 3

Experimental objective: feasibility confirmation and screening of different types of polyvinyl alcohol (0.4%) aqueous solutions.

Process conditions: the reactor model was -1#, and the volume of the continuous phase was 3 L.

Solvents of the dispersed phase: ethyl acetate, ethanol (absolute), and water, in a mass ratio of 5.4 : 3.2 : 1 (62 g in total).

Solutes of the dispersed phase: API, EPO, N10, magnesium oxide, triethyl citrate, and titanium dioxide, in a mass ratio of 5.6 : 15 : 37 : 15 : 1 : 1 (7.46 g in total).

Continuous phase: different types of 0.4% polyvinyl alcohol series aqueous solutions, continuous phase temperature: normal temperature, and continuous phase pH: 7.

Preparation process: the same as Example 1.

The indexes of the prepared taste-masking microspheres are shown in Table 3.

**Table 3**

| Test batch No. | Polyvinyl alcohol type | Yield (%) | Content (%) | Encapsulation efficiency (%) | Dissolution rate after 30 min (%) |
|---|---|---|---|---|---|
| FC421-3 | 03-88 | 90.5 | 6.2 | 75.5 | 99.5 |
| FC422-1 | 05-88 | 90.5 | 6.6 | 80.1 | 96.8 |
| FC422-2 | 17-88 | 90.9 | 6.0 | 72.6 | 97.2 |
| FC421-5 | 20-88 | 94.6 | 5.8 | 72.3 | 96.6 |
| FC421-4 | 25-88 | 87.2 | 6.4 | 74.6 | 98.5 |

### Example 4

Experimental objective: feasibility scope determination and screening and optimization of polyvinyl alcohol (05-88) aqueous solutions with different concentrations.

Process conditions: the reactor model was -1#, and the volume of the continuous phase was 3 L.

Solvents of the dispersed phase: ethyl acetate, ethanol (absolute), and water, in a mass ratio of 5.4 : 3.2 : 1 (62 g in total).

Solutes of the dispersed phase: API, EPO, N10, magnesium oxide, titanium dioxide, and triethyl citrate, in a mass ratio of 5.6 : 15 : 37 : 15 : 1 : 1 (7.46 g in total).

Continuous phase: different concentrations of polyvinyl alcohol (05-88) series aqueous solutions, continuous phase temperature: normal temperature, and continuous phase pH: 7.

Preparation process: with reference to the basic example, the number of the feed ports was 1 each side, for a total of 3; the feeding was carried out with stirring at 120 rpm for 20 min (peristaltic pump model: Signal 2S, feeding rotational speed: 4 rpm); and after feeding, the stirring speed was 80 rpm, and the stirring time was 10 min.

The indexes of the prepared taste-masking microspheres are shown in Table 4.

**Table 4**

| Test batch No. | Polyvinyl alcohol concentration (%) | Yield (%) | Content (%) | Encapsulatio n efficiency (%) | Dissolution rate after 30 min (%) |
|---|---|---|---|---|---|
| FC422-3 | 0.05 | 87.6 | 6.2 | 71.8 | 98.8 |
| FC422-4 | 0.4 | 84.7 | 6.6 | 72.9 | 98.3 |
| FC422-7 | 0.8 | 86.0 | 5.9 | 67.1 | 96.8 |
| FC422-11 | 2.0 | 84.4 | 5.7 | 64.0 | 97.0 |

### Example 5

Experimental objective: feasibility study scope determination and screening and optimization of sodium oleate aqueous solutions with different concentrations.

Process conditions: the reactor model was -1#, and the volume of the continuous phase was 3 L.

Solvents of the dispersed phase: ethyl acetate, ethanol (absolute), and water, in a mass ratio of 5.4 : 3.2 : 1 (62 g in total).

Solutes of the dispersed phase: API, EPO, N10, magnesium oxide, triethyl citrate, magnesium stearate, silica, titanium dioxide, and talc powder, in a mass ratio of 5.6 : 15 : 37 : 15 : 5 : 2 : 2 : 1 : 1 (8.36 g in total).

Continuous phase: different low concentrations of sodium oleate series aqueous solutions, continuous phase temperature: normal temperature, and continuous phase pH: 7.

Preparation process: the same as Example 1.

The indexes of the prepared taste-masking microspheres are shown in Table 5.

**Table 5**

| Test batch No. | Sodium oleate concentration (%) | Yield (%) | Content (%) | Encapsulation efficiency (%) | Dissolution rate after 30 min (%) |
|---|---|---|---|---|---|
| FC620-1 | 0.05 | 83.4 | 4.7 | 60.0 | 96.2 |
| FC620-2 | 0.025 | 88.7 | 4.4 | 60.5 | 95.6 |
| FC620-3 | 0.01 | 83.4 | 4.9 | 61.0 | 98.4 |

### Example 6

Experimental objective: feasibility study scope determination and optimization of SDS (sodium dodecyl sulfate) aqueous solutions with different concentrations.

Process conditions: the reactor model was -1#, and the volume of the continuous phase was 3 L.

Solvents of the dispersed phase: ethyl acetate, ethanol (absolute), and water, in a mass ratio of 5.4 : 3.2 : 1 (62 g in total).

Solutes of the dispersed phase: API, EPO, N10, magnesium oxide, triethyl citrate, magnesium stearate, silica, titanium dioxide, and talc powder, in a mass ratio of 5.6 : 15 : 37 : 15 : 5 : 2 : 2 : 1 : 1 (8.36 g in total).

Continuous phase: different concentrations of SDS series aqueous solutions, continuous phase temperature: normal temperature, and continuous phase pH: 7.

Preparation process: with reference to the basic example, the number of the feed ports was 1 each side, for a total of 3; the feeding was carried out with stirring at 180 rpm for 10 min (peristaltic pump model: Signal 2S, feeding rotational speed: 15 rpm); and after feeding, the stirring speed was 120 rpm, and the stirring time was 60 min.

The indexes of the prepared taste-masking microspheres are shown in Table 6.

**Table 6**

| Test batch No. | SDS concentration (%) | Yield (%) | Content (%) | Encapsulation efficiency (%) | Dissolution rate after 30 min (%) |
|---|---|---|---|---|---|
| FC713-1 | 0.02 | 86.8 | 5.9 | 76.4 | 95.2 |
| FC421-2 | 0.05 | 88.4 | 6.0 | 79.5 | 99.8 |
| FC325-3 | 0.2 | 82.1 | 5.1 | 61.8 | 99.5 |

### Example 7

Experimental objective: feasibility study scope determination and optimization of CMC-Na (sodium carboxymethyl cellulose) aqueous solutions with different concentrations.

Process conditions: the reactor model was -1#, and the volume of the continuous phase was 3 L.

Solvents of the dispersed phase: ethyl acetate, ethanol (absolute), and water, in a mass ratio of 5.4 : 3.2 : 1 (62 g in total).

Solutes of the dispersed phase: API, EPO, N10, magnesium oxide, triethyl citrate, silica, povidone, titanium dioxide, and talc powder, in a mass ratio of 5.6 : 15 : 37 : 15 : 5 : 2 : 2 : 1 : 1 (8.36 g in total).

Continuous phase: different concentrations of CMC-Na series aqueous solutions, continuous phase temperature: normal temperature, and continuous phase pH: 7.

Preparation process: the same as Example 1.

The indexes of the prepared taste-masking microspheres are shown in Table 7.

**Table 7**

| Test batch No. | CMC-Na concentration (%) | Yield (%) | Content (%) | Encapsulatio n efficiency (%) | Dissolution rate after 30 min (%) |
|---|---|---|---|---|---|
| FC422-5 | 0.02 | 84.6 | 5.9 | 74.9 | 100.3 |
| FC325-2 | 0.05 | 84.9 | 6.6 | 78.6 | 101.3 |
| FC422-6 | 0.25 | 91.6 | 5.5 | 76.5 | 98.4 |

### Example 8

Experimental objective: feasibility confirmation of a surfactant-free continuous phase.

Process conditions: the reactor model was -1#, and the volume of the continuous phase was 3 L.

Solvents of the dispersed phase: ethyl acetate, ethanol (absolute), and water, in a mass ratio of 5.4 : 3.2 : 1 (62 g in total).

Solutes of the dispersed phase: API, EPO, N10, magnesium oxide, triethyl citrate, and titanium dioxide, in a mass ratio of 5.6 : 15 : 37 : 15 : 5 : 1 (7.86 g in total).

Continuous phase: the continuous phases were shown in Table 8. Continuous phase temperature: normal temperature, and continuous phase pH: 7.

Preparation process: the same as Example 1.

The indexes of the prepared taste-masking microspheres are shown in Table 8.

**Table 8**

| Test batch No. | Continuous phase | Yield (%) | Conten t (%) | Encapsulation efficiency (%) | Dissolution rate after 30 min (%) |
|---|---|---|---|---|---|
| FC424-3 | Purified water | 88.1 | 6.5 | 79.9 | 99.6 |
| FC424-2 | 50% ethanol aqueous solution | 87.2 | 5.8 | 71.9 | 100.1 |
| FC424-11 | 10% ethyl acetate aqueous solution | 86.5 | 5.5 | 65.3 | 98.0 |
| FC517-2 | 2% dichloromethane aqueous solution | 84.2 | 5.8 | 67.7 | 99.4 |

### Example 9

Experimental objective: feasibility scope determination and screening and optimization of 0.4% polyvinyl alcohol aqueous solutions at different test temperatures.

Process conditions: the reactor model was -1#, and the volume of the continuous phase was 3 L.

Solvents of the dispersed phase: ethyl acetate, ethanol (absolute), and water, in a mass ratio of 5.4 : 3.2 : 1 (62 g in total).

Solutes of the dispersed phase: API, E100, N10, magnesium oxide, triethyl citrate, povidone, silica, talc powder, and titanium dioxide, in a mass ratio of 5.6 : 15 : 37 : 15 : 5 : 2 : 2 : 1 : 1 (8.36 g in total).

Continuous phase: 0.4% polyvinyl alcohol (05-88) aqueous solutions, continuous phase temperature: temperatures as shown in Table 9, continuous phase pH: 7.

Preparation process: the same as Example 1.

The indexes of the prepared taste-masking microspheres are shown in Table 9.

**Table 9**

| Test batch No. | Continuous phase temperature (°C) | Yield (%) | Content (%) | Encapsulation efficiency (%) | Dissolution rate after 30 min (%) |
|---|---|---|---|---|---|
| FC424-8 | 2.0 | 95.9 | 6.4 | 90.1 | 95.5 |
| FC424-5 | 8.1 | 87.2 | 6.9 | 90.0 | 99.7 |
| FC424-6 | 10.7 | 93.6 | 6.4 | 90.2 | 99.4 |
| FC424-7 | 17.1 | 85.0 | 5.9 | 76.7 | 93.9 |
| FC424-9 | 30.3 | 88.7 | 4.9 | 65.9 | 93.4 |

### Example 10

Experimental objective: feasibility scope determination and optimization of processes reusing different proportions of the continuous phase.

Process conditions: the reactor model was -1#, and the volume of the continuous phase was 3 L.

Solvents of the dispersed phase: ethyl acetate, ethanol (absolute), and water, in a mass ratio of 5.4 : 3.2 : 1 (62 g in total).

Solutes of the dispersed phase: API, EPO, N10, magnesium oxide, triethyl citrate, and titanium dioxide, in a mass ratio of 5.6 : 15 : 37 : 15 : 5 : 1 (7.86 g in total).

Continuous phase: reusing 0.4% polyvinyl alcohol (05-88) aqueous solutions in proportions as shown in Table 10, continuous phase temperature: normal temperature, and continuous phase pH: 7.

Preparation process: the same as Example 1.

The indexes of the prepared taste-masking microspheres are shown in Table 10.

**Table 10**

| Test batch No. | Reuse proportion of continuous phase (%) | Yield (%) | Content (%) | Encapsulation efficiency (%) | Dissolution rate after 30 min (%) |
|---|---|---|---|---|---|
| FC425-3 | 10 | 82.4 | 5.8 | 68.8 | 97.3 |
| FC425-2 | 30 | 86.7 | 6.4 | 77.7 | 99.7 |
| FC425-1 | 50 | 87.0 | 5.9 | 73.2 | 99.7 |
| FC424-10 | 80 | 87.2 | 5.6 | 68.0 | 98.1 |

### Example 11

Experimental objective: feasibility scope determination and optimization of 0.4% polyvinyl alcohol aqueous solutions controlled at different pH.

Process conditions: the reactor model was -1#, and the volume of the continuous phase was 3 L.

Solvents of the dispersed phase: ethyl acetate, ethanol (absolute), and water, in a mass ratio of 5.4 : 3.2 : 1 (62 g in total).

Solutes of the dispersed phase: API, E100, N10, magnesium oxide, triethyl citrate, povidone, magnesium stearate, talc powder, and titanium dioxide, in a mass ratio of 5.6 : 15 : 37 : 15 : 5 : 2 : 2 : 1 : 1 (8.36 g in total).

Continuous phase: 0.4% polyvinyl alcohol (05-88) aqueous solutions, continuous phase temperature: normal temperature, and continuous phase pH: as shown in Table 11.

Preparation process: the same as Example 1.

The indexes of the prepared taste-masking microspheres are shown in Table 11.

**Table 11**

| Test batch No. | pH control of continuous phase | | Yield (%) | Content (%) | Encapsulation efficiency (%) | Dissolution rate after 30 min (%) |
|---|---|---|---|---|---|---|
| | Initial value | Endpoint value | | | | |
| FC324-1 | 6.14 | 9.63 | 85.3 | 6.0 | 75.4 | 100.4 |
| FC324-5 | 6.98 | 9.37 | 91.9 | 6.7 | 92.8 | 100.5 |
| FC324-2 | 8.10 | 9.57 | 78.4 | 6.8 | 79.0 | 100.4 |
| FC324-3 | 9.01 | 9.53 | 86.9 | 6.3 | 82.0 | 100.5 |
| FC324-4 | 11.01 | 9.70 | 90.4 | 7.0 | 94.6 | 100.9 |

### Example 12

Experimental objective: feasibility scope determination and screening of processes with different ratios of ethyl acetate and ethanol.

Process conditions: the reactor model was -1#, and the volume of the continuous phase was 3 L.

Solvents of the dispersed phase: ethyl acetate + ethanol, 65 mL in total, added with 6.5 mL of water.

Solutes of the dispersed phase: API, EPO, N10, and magnesium oxide, in a mass ratio of 1 : 2.68 : 6.61 : 2.68 (7.26 g in total).

Continuous phase: 0.4% polyvinyl alcohol (05-88) aqueous solutions, continuous phase temperature: normal temperature, and continuous phase pH: 7.

Preparation process: the same as Example 1.

The indexes of the prepared taste-masking microspheres are shown in Table 12.

**Table 12**

| Test batch No. | Volume ratio of ethyl acetate/ethanol | Yield (%) | Conten t (%) | Encapsulation efficiency (%) | Dissolution rate after 30 min (%) |
|---|---|---|---|---|---|
| FC330-3 | 4 : 1 | 93.7 | 7.2 | 87.1 | 93.1 |
| FC328-2 | 2:1 | 82.6 | 7.1 | 76.3 | 84.7 |
| FC328-1 | 1.5 : 1 | 85.4 | 6.7 | 75.1 | 86.7 |
| FC328-4 | 1:1 | 93.1 | 6.6 | 79.2 | 98.1 |

### Example 13

Experimental objective: feasibility scope determination and screening of processes (pH-independent carrier) with different ratios of ethyl acetate and ethanol.

Process conditions: the reactor model was -1#, and the volume of the continuous phase was 3 L.

Solvents of the dispersed phase: ethyl acetate + ethanol, 66 mL in total.

Solutes of the dispersed phase: API, RSPO, RLPO, N10, fumaric acid, succinic acid, BHT, titanium dioxide, and TEC, in a mass ratio of 1 : 0.6 : 2.4 : 6 : 0.6 : 0.1 : 0.02 : 0.05 : 0.2 (5.48 g in total).

Continuous phase: 0.4% polyvinyl alcohol (05-88) aqueous solutions, continuous phase temperature: low temperature (5°C), and continuous phase pH: 6.

Preparation process: the same as Example 1.

The indexes of the prepared taste-masking microspheres are shown in Table 13.

**Table 13**

| Test batch No. | Volume ratio of ethyl acetate/ethanol | Yield (%) | Content (%) | Encapsulation efficiency (%) | Dissolution rate after 30 min (%) |
|---|---|---|---|---|---|
| FCC406-1 | 5:1 | 79.5 | 8.9 | 77.6 | 96.4 |
| FCC406-3 | 10 : 1 | 81.0 | 9.2 | 81.8 | 86.0 |
| FCC406-4 | 20 : 1 | 78.3 | 10.4 | 88.5 | 90.7 |
| FCC406-2 | 25 : 1 | 81.4 | 9.6 | 85.1 | 97.3 |

### Example 14

Experimental objective: feasibility confirmation of processes using mixed solvents of two different phases.

Process conditions: the reactor model was -1#, and the volume of the continuous phase was 3 L.

Solvents of the dispersed phase: two-phase mixed solvents (see Table 14) with a volume ratio of 1.5 : 1, 65 mL in total, with 6.5 mL of water.

Solutes of the dispersed phase: API, EPO, N10, and magnesium oxide, in a mass ratio of 1 : 2.68 : 6.61 : 2.68 (7.26 g in total).

Continuous phase: 0.4% polyvinyl alcohol (05-88) aqueous solutions, continuous phase temperature: normal temperature, and continuous phase pH: 7.

Preparation process: the same as Example 1.

The indexes of the prepared taste-masking microspheres are shown in Table 14.

**Table 14**

| Test batch No. | Two-phase mixed solvent | Yield (%) | Content (%) | Encapsulatio n efficiency (%) | Dissolution rate after 30 min (%) |
|---|---|---|---|---|---|
| FC322-1 | n-Butanol/ethanol | 84.5 | 6.1 | 67.3 | 99.5 |
| FC426-2 | Ethyl acetate/methanol | 87.5 | 7.1 | 79.6 | 93.2 |
| FC321-2 | Ethyl acetate/isopropanol | 90.9 | 7.3 | 86.8 | 98.3 |
| FC321-3 | Ethyl acetate/acetone | 91.5 | 7.7 | 93.2 | 87.7 |
| FC427-2 | Ethyl acetate/acetonitrile | 94.7 | 6.5 | 80.9 | 101.0 |
| FC323-4 | Dichloromethane/ethanol | 82.9 | 5.9 | 63.4 | 100.9 |

### Example 15

Experimental objective: feasibility scope determination and screening and optimization of processes by evaluation with different proportions of water added.

Process conditions: the reactor model was -1#, and the volume of the continuous phase was 3 L.

Solvents of the dispersed phase: ethyl acetate, and ethanol (absolute), in a mass ratio of 1.69 : 1 (55.5 g in total), with water added according to the prescribed addition amounts in Table 15.

Solutes of the dispersed phase: API, EPO, N10, magnesium oxide, triethyl citrate, and titanium dioxide, in a mass ratio of 5.6 : 15 : 37 : 15 : 5 : 1 (7.86 g in total).

Continuous phase: 0.4% polyvinyl alcohol (05-88) aqueous solutions, continuous phase temperature: normal temperature, and continuous phase pH: 7.

Preparation process: the same as Example 1.

The indexes of the prepared taste-masking microspheres are shown in Table 15.

**Table 15**

| Test batch No. | Prescribe d addition amount of water (mL) | Mass percentage of water in dispersed phase liquid (%) | Correspon ding content of aqueous ethanol 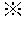 (%) | Yield (%) | Conte nt (%) | Encaps ulation efficien cy (%) | Dissolutio n rate after 30 min (%) |
|---|---|---|---|---|---|---|---|
| FC712-2 | 2.0 | 3.5 | 91.1 | 84.9 | 4.6 | 53.0 | 97.9 |
| FC427-6 | 4.5 | 7.5 | 82.0 | 85.0 | 6.9 | 80.9 | 96.6 |
| FC427-7 | 6.5 | 10.5 | 75.9 | 85.8 | 6.3 | 75.4 | 96.8 |
| FC427-8 | 10 | 15.2 | 67.2 | 84.2 | 6.4 | 74.7 | 98.4 |
| FC427-11 | 20 | 26.4 | 50.6 | 77.1 | 5.6 | 60.6 | 100.0 |
| FC429-4 | 28 | 33.5 | 42.3 | 79.8 | 5.4 | 60.5 | 83.5 |

| | | | | | | | |
|---|---|---|---|---|---|---|---|
| Note: 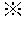 The corresponding percentage content of aqueous ethanol refers to the minimum ethanol content required to achieve the prescribed ratio by adding aqueous ethanol (which can reduce the procurement and recovery costs of ethanol) according to the prescribed ratio of ethyl acetate, ethanol, and water. For instance, if 6.5 mL of water is required according to the prescription, the aqueous (or recovered) ethanol correspondingly added must have a mass concentration of 75.9% or more, and if the newly purchased or recovered ethanol has a mass concentration of 80%, the 80% charge amount can be calculated based on the ratio of absolute ethanol, followed by supplementing water in a difference amount calculated according to the process ratio. | | | | | | | |

### Example 16

Experimental objective: feasibility confirmation of processes by evaluating water-free dispersed phase solvents.

Process conditions: the reactor model was -1#, and the volume of the continuous phase was 3 L.

Solvents of the dispersed phase: ethyl acetate, and ethanol (absolute), in a mass ratio of 4.56 : 1 (57.1 g in total).

Solutes of the dispersed phase: API, EPO, N10, magnesium oxide, triethyl citrate, and titanium dioxide, in a mass ratio of 5.6 : 22 : 37 : 15 : 7 : 1 (8.76 g in total).

Continuous phase: 0.2% Tween 80 aqueous solutions, continuous phase temperature: low temperature (3.5°C), and continuous phase pH: 7.

Preparation process: the same as Example 1.

The indexes of the prepared taste-masking microspheres are shown in Table 16.

**Table 16**

| Test batch No. | Yield (%) | Content (%) | Encapsulation efficiency (%) | Dissolution rate after 30 min (%) |
|---|---|---|---|---|
| FC726-3 | 67.1 | 5.9 | 61.6 | 87.0 |
| FC726-4 | 69.9 | 5.7 | 62.9 | 88.6 |

### Example 17

Experimental objective: feasibility confirmation of processes using mixed solvents of three different solvents.

Process conditions: the reactor model was -1#, and the volume of the continuous phase was 3 L.

Solvents of the dispersed phase: three-solvent mixed solvents (see Table 17 ), with a volume ratio of 1.5 : 0.5 : 0.5, 65 mL in total, with 6.5 mL of water.

Solutes of the dispersed phase: API, EPO, N10, and magnesium oxide, in a mass ratio of 1 : 2.68 : 6.61 : 2.68 (7.26 g in total).

Continuous phase: 0.4% polyvinyl alcohol (05-88) aqueous solutions, continuous phase temperature: normal temperature, and continuous phase pH: 7.

Preparation process: the same as Example 1.

The indexes of the prepared taste-masking microspheres are shown in Table 17.

**Table 17**

| Test batch No. | Three-solvent mixed solvent | Yield (%) | Conten t (%) | Encapsula tion efficiency (%) | Dissolution rate after 30 min (%) |
|---|---|---|---|---|---|
| FC428-2 | Ethyl acetate/ethanol/n-butanol | 88.2 | 5.5 | 64.7 | 91.5 |
| FC428-5 | Ethyl acetate/ethanol/methanol | 92.1 | 6.0 | 72.5 | 96.1 |
| FC428-8 | Ethyl acetate/ethanol/isopropanol | 86.7 | 5.5 | 61.8 | 97.2 |
| FC429-1 | Ethyl acetate/ethanol/acetone | 86.5 | 5.7 | 64.6 | 97.4 |

### Example 18

Experimental objective: feasibility determination and optimization of processes with different types of pH-dependent carriers.

Process conditions: the reactor model was -1#, and the volume of the continuous phase was 3 L.

Solvents of the dispersed phase: ethyl acetate, ethanol (absolute), and water, in a mass ratio of 7.8 : 3.9 : 1 (57.1 g in total).

Solutes of the dispersed phase: API, different types of carriers, N10, magnesium oxide, and triethyl citrate, in a mass ratio of 1.12 : 3 : 9.24 : 1.16 : 1 (7.76 g in total). The types of carriers are shown in Table 18.

Continuous phase: 0.4% polyvinyl alcohol (05-88) solutions, continuous phase temperature: 9°C, and continuous phase pH: 7.

Preparation process: the same as Example 1.

The indexes of the prepared taste-masking microspheres are shown in Table 18.

**Table 18**

| Test batch No. | pH-dependent carrier | Yield (%) | Content (%) | Encapsulation efficiency (%) | Dissolution rate after 30 min (%) |
|---|---|---|---|---|---|
| FC608-1 | EPO | 85.6 | 5.7 | 68.6 | 99.7 |
| FC608-2 | E100 | 85.1 | 5.5 | 64.6 | 99.1 |
| FC608-3 | 30D100P | 85.5 | 6.0 | 71.0 | 94.3 |
| FC616-4 | AEA | 89.7 | 4.7 | 60.2 | 99.9 |

### Example 19

Experimental objective: feasibility determination and optimization of processes with different types of pH-independent carriers.

Process conditions: the reactor model was -1#, and the volume of the continuous phase was 3 L.

Solvents of the dispersed phase: ethyl acetate, and ethanol (absolute), in a mass ratio of 20 : 1 (59 g in total).

Solutes of the dispersed phase: API, different types of carriers, N10, fumaric acid, succinic acid, BHT, titanium dioxide, and TEC, in a mass ratio of 1 : 3 : 6 : 0.6 : 0.1 : 0.02 : 0.05 : 0.2 (5.48 g in total). The types of carriers are shown in Table 19.

Continuous phase: 0.4% polyvinyl alcohol (05-88) solutions, continuous phase temperature: low temperature (5°C), and continuous phase pH: 6.

Preparation process: the same as Example 1.

The indexes of the prepared taste-masking microspheres are shown in Table 19.

**Table 19**

| Test batch No. | pH-independent carrier | Yield (%) | Content (%) | Encapsulation efficiency (%) | Dissolution rate after 30 min (%) |
|---|---|---|---|---|---|
| FCC328-1 | RLPO | 86.4 | 8.4 | 79.2 | 84.5 |
| FCC328-3 | RL100 | 85.2 | 9.3 | 86.0 | 80.3 |
| FCC328-2 | RSPO | 89.6 | 8.0 | 78.9 | 95.8 |
| FCC328-4 | RS100 | 87.3 | 7.6 | 73.4 | 87.7 |

### Example 20

Experimental objective: feasibility determination and optimization of processes with combinations of different types of pH-independent carriers.

Process conditions: the reactor model was -1#, and the volume of the continuous phase was 3 L.

Solvents of the dispersed phase: ethyl acetate, and ethanol (absolute), in a mass ratio of 20 : 1 (59 g in total).

Solutes of the dispersed phase: API, different types of carriers, N10, fumaric acid, succinic acid, BHT, titanium dioxide, and TEC, in a mass ratio of 1 : 3 : 6 : 0.6 : 0.1 : 0.02 : 0.05 : 0.2 (5.48 g in total). The types of carriers are shown in Table 20.

Continuous phase: 0.4% polyvinyl alcohol (05-88) solutions, continuous phase temperature: low temperature (6°C), and continuous phase pH: 6.

Preparation process: the same as Example 1.

The indexes of the prepared taste-masking microspheres are shown in Table 20.

**Table 20**

| Test batch No. | Ratio of RSPO : RLPO | Yield (%) | Content (%) | Encapsulatio n efficiency (%) | Dissolution rate after 30 min (%) |
|---|---|---|---|---|---|
| FCC329-1 | 1 : 1 | 88.2 | 7.2 | 69.0 | 92.5 |
| FCC329-2 | 1:4 | 87.9 | 7.8 | 74.5 | 90.0 |
| FCC329-3 | 1: 6 | 88.9 | 7.3 | 70.7 | 98.9 |
| FCC329-4 | 1:8 | 85.3 | 8.7 | 81.3 | 89.8 |

### Example 21

Experimental objective: feasibility scope determination and screening of processes with different proportions of pH-dependent carrier (EP0) and matrix-type carrier (N10).

Process conditions: the reactor model was -1#, and the volume of the continuous phase was 3 L.

Solvents of the dispersed phase: ethyl acetate, ethanol (absolute), and water, in a mass ratio of 5.4 : 3.2 : 1 (62 g in total).

Solutes of the dispersed phase: API and magnesium oxide, in a mass ratio of 1 : 2.68 (feeding amount: 2.06 g), and EPO and N10 added according to the feeding percentage in Table 21 (total feeding amount of solutes: 7.26 g).

Continuous phase: 0.4% polyvinyl alcohol (05-88) aqueous solutions, continuous phase temperature: normal temperature, and continuous phase pH: 7.

Preparation process: the same as Example 1.

The indexes of the prepared taste-masking microspheres are shown in Table 21.

**Table 21**

| Test batch No. | Feeding percentage of EPO in solutes (%) | Feeding percentage of N10 in solutes (%) | Yield (%) | Content (%) | Encapsula tion efficiency (%) | Dissolution rate after 30 min (%) |
|---|---|---|---|---|---|---|
| FC429-3 | 5 | 66.5 | 90.9 | 6.3 | 75.8 | 60.1 |
| FC429-5 | 10 | 61.6 | 89.0 | 6.4 | 75.6 | 89.3 |
| FC429-7 | 20 | 51.6 | 92.8 | 6.6 | 78.7 | 98.7 |
| FC429-11 | 40 | 31.6 | 87.5 | 5.9 | 68.2 | 97.6 |

### Example 22

Experimental objective: feasibility determination and optimization of processes with different types of N-series matrix-type carriers and a pH-dependent carrier.

Process conditions: the reactor model was -1#, and the volume of the continuous phase was 3 L.

Solvents of the dispersed phase: ethyl acetate, ethanol (absolute), and water, in a mass ratio of 5.4 : 3.2 : 1 (62 g in total).

Solutes of the dispersed phase: API, EPO, and magnesium oxide, in a mass ratio of 1 : 2.68 : 2.68 (3.56 g in total), and different types of ethylcellulose in feeding amounts as shown in Table 22.

Continuous phase: 0.4% polyvinyl alcohol (05-88) aqueous solutions, continuous phase temperature: normal temperature, and continuous phase pH: 7.

Preparation process: the same as Example 1.

The indexes of the prepared taste-masking microspheres are shown in Table 22.

**Table 22**

| Test batch No. | Ethylcellul ose type | Feeding amount of ethylcellulose (g) | Yield (%) | Content (%) | Encapsulation efficiency (%) | Dissolution rate after 30 min (%) |
|---|---|---|---|---|---|---|
| FC520-2 | N7 | 3.7 | 90.8 | 6.9 | 81.2 | 98.4 |
| FC317-1 | N10 | 3.7 | 93.5 | 6.8 | 83.0 | 100.2 |
| FC326-4 | N22 | 3.0 | 80.8 | 7.0 | 66.6 | 90.5 |
| FC326-5 | N50 | 2.7 | 72.3 | 8.5 | 68.8 | 93.6 |

### Example 23

Experimental objective: feasibility determination and optimization of processes with different types of N-series matrix-type carriers and a pH-independent carrier.

Process conditions: the reactor model was -1#, and the volume of the continuous phase was 3 L.

Solvents of the dispersed phase: ethyl acetate, and ethanol (absolute), in a mass ratio of 20 : 1 (59 g in total).

Solutes of the dispersed phase: API, RSPO, RLPO, ethylcellulose types, fumaric acid, succinic acid, BHT, titanium dioxide, and TEC, in a mass ratio of 1 : 0.6 : 2.4 : 6 : 0.6 : 0.1 : 0.02 : 0.05 : 0.2 (5.48 g in total). The types of the matrix carriers are shown in Table 23.

Continuous phase: 0.4% polyvinyl alcohol (05-88) aqueous solutions, continuous phase temperature: low temperature (5°C), and continuous phase pH: 6.

Preparation process: the same as Example 1.

The indexes of the prepared taste-masking microspheres are shown in Table 23.

**Table 23**

| Test batch No. | Ethylcellulose type | Yield (%) | Content (%) | Encapsulation efficiency (%) | Dissolution rate after 30 min (%) |
|---|---|---|---|---|---|
| FCC330-1 | N7 | 78.6 | 9.7 | 83.9 | 84.6 |
| FCC330-3 | N10 | 79.5 | 9.7 | 84.3 | 90.3 |
| FCC330-4 | N22 | 80.7 | 9.6 | 83.4 | 90.2 |
| FCC330-2 | N50 | 80.6 | 9.0 | 79.4 | 86.4 |

### Example 24

Experimental objective: feasibility scope determination and optimization and screening of processes by evaluating different addition percentages of magnesium oxide.

Process conditions: the reactor model was -1#, and the volume of the continuous phase was 3 L.

Solvents of the dispersed phase: ethyl acetate, ethanol (absolute), and water, in a mass ratio of 5.4 : 3.2 : 1 (62 g in total).

Solutes of the dispersed phase: API and E100, in a mass ratio of 1 : 2.68 (feed amount: 2.06 g), and magnesium oxide added in the proportions in Table 24 (total feeding amount of solutes: 7.26 g, with the sum of the amounts of N7 and magnesium oxide being 5.2 g).

Continuous phase: 0.4% polyvinyl alcohol (05-88) aqueous solutions, continuous phase temperature: normal temperature, and continuous phase pH: 7.

Preparation process: the same as Example 1.

The indexes of the prepared taste-masking microspheres are shown in Table 24.

**Table 24**

| Test batch No. | Proportion of magnesium oxide added in solutes (%) | Yield (%) | Content (%) | Encapsulatio n efficiency (%) | Dissolution rate after 30 min (%) |
|---|---|---|---|---|---|
| FC627-1 | 2.1 | 91.6 | 5.9 | 70.1 | 100.8 |
| FC509-2 | 6.1 | 88.6 | 6.6 | 74.5 | 94.0 |
| FC509-3 | 8.1 | 91.5 | 6.9 | 80.9 | 94.8 |
| FC506-6 | 20.6 | 89.0 | 6.1 | 69.0 | 97.5 |
| FC506-8 | 30.3 | 89.7 | 6.1 | 70.6 | 97.9 |

### Example 25

Experimental objective: feasibility scope determination and optimization of processes by evaluation with different stirring time after the addition of magnesium oxide.

Process conditions: the reactor model was -1#, and the volume of the continuous phase was 3 L.

Solvents of the dispersed phase: ethyl acetate, ethanol (absolute), and water, in a mass ratio of 5.4 : 3.2 : 1 (62 g in total).

Solutes of the dispersed phase: API, E100, N7, and magnesium oxide, in a mass ratio of 1 : 2.68 : 6.61 : 2.68 (7.26 g in total).

The stirring time after the addition of magnesium oxide was controlled according to Table 25.

Continuous phase: 0.4% polyvinyl alcohol (05-88) aqueous solutions, continuous phase temperature: normal temperature, and continuous phase pH: 7.

Preparation process: the same as Example 1.

The indexes of the prepared taste-masking microspheres are shown in Table 25.

**Table 25**

| Test batch No. | Stirring time after addition of magnesium oxide (h) | Yield (%) | Content (%) | Encapsulation efficiency (%) | Dissolution rate after 30 min (%) |
|---|---|---|---|---|---|
| FC507-2 | 0 | 90.2 | 6.2 | 72.2 | 100.0 |
| FC507-3 | 0.66 | 92.3 | 6.2 | 72.7 | 98.1 |
| FC507-5 | 2 | 90.2 | 6.3 | 73.8 | 98.8 |
| FC507-1 | 8 | 89.8 | 6.5 | 75.3 | 97.6 |

As can be seen from Table 25, the stirring time after the addition of magnesium oxide has little effect on the yield, content, encapsulation efficiency and dissolution rate of products.

### Example 26

Experimental objective: feasibility determination and optimization of processes by evaluation with different types of alkalizing agents.

Process conditions: the reactor model was -1#, and the volume of the continuous phase was 3 L. Stirring form: stirring with a three-layer push-down paddle.

Solvents of the dispersed phase: ethyl acetate, ethanol (absolute), and water, in a mass ratio of 5.4 : 3.2 : 1 (62 g in total).

Solutes of the dispersed phase: API, EPO, N10, alkalizing agents, triethyl citrate, silica, povidone, titanium dioxide, and talc powder, in a mass ratio of 5.6 : 15 : 37 : 15 : 5 : 2 : 2 : 1 : 1 (8.36 g in total), with the types of alkalizing agents shown in Table 26.

Continuous phase: 0.4% polyvinyl alcohol (05-88) aqueous solutions, continuous phase temperature: normal temperature, and continuous phase pH: 7.

Preparation process: the same as Example 1.

The indexes of the prepared taste-masking microspheres are shown in Table 26.

**Table 26**

| Test batch No. | Alkalizing agent type | Yield (%) | Content (%) | Encapsulation efficiency (%) | Dissolution rate after 30 min (%) |
|---|---|---|---|---|---|
| FC319-1 | Meglumine | 62.7 | 7.1 | 66.8 | 101.4 |
| FC319-2 | Tromethami ne | 61.3 | 7.4 | 66.7 | 102.7 |
| FC318-3 | Sodium bicarbonate | 70.1 | 7.2 | 75.8 | 103.9 |
| FC318-2 | Sodium carbonate | 70.3 | 5.7 | 60.1 | 99.7 |

### Example 27

Experimental objective: Feasibility determination of processes by evaluation with no alkalizing agent added.

Process conditions: the reactor model was -1#, and the volume of the continuous phase was 3 L.

Solvents of the dispersed phase: ethyl acetate, ethanol (absolute), and water, in a mass ratio of 7.2 : 1.58 : 1 (63.6 g in total).

Solutes of the dispersed phase: API, EPO, N10, triethyl citrate, and titanium dioxide, in a mass ratio of 5.6 : 18 : 53 : 5 : 1 (feeding amount: 8.26 g).

Continuous phase: 0.4% polyvinyl alcohol (05-88) aqueous solutions, continuous phase temperature: low temperature (4.5°C), and continuous phase pH: 7.

Preparation process: the same as Example 1.

The indexes of the prepared taste-masking microspheres are shown in Table 27.

**Table 27**

| Test batch No. | Yield (%) | Content (%) | Encapsulation efficiency (%) | Dissolution rate after 30 min (%) |
|---|---|---|---|---|
| FC726-1 | 77.2 | 5.4 | 61.9 | 90.1 |
| FC726-2 | 78.2 | 5.5 | 63.9 | 91.8 |

### Example 28

Experimental objective: feasibility determination and optimization of processes by evaluation with different types of opacifiers.

Process conditions: the reactor model was -1#, and the volume of the continuous phase was 3 L.

Solvents of the dispersed phase: ethyl acetate, ethanol (absolute), and water, in a mass ratio of 5.4 : 3.2 : 1 (62 g in total).

Solutes of the dispersed phase: API, EPO, N10, magnesium oxide, and opacifiers, in a mass ratio of 5.6 : 15 : 40 : 10 : 1 (7.2 g in total), with the types of opacifiers shown in Table 28.

Continuous phase: 0.4% polyvinyl alcohol (05-88) aqueous solutions, continuous phase temperature: normal temperature, and continuous phase pH: 7.

Preparation process: the same as Example 1.

The indexes of the prepared taste-masking microspheres are shown in Table 28.

**Table 28**

| Test batch No. | Opacifier type | Yield (%) | Content (%) | Encapsulatio n efficiency (%) | Dissolution rate after 30 min (%) |
|---|---|---|---|---|---|
| FC509-4 | Zinc oxide | 96.7 | 6.6 | 82.8 | 97.2 |
| FC407-1 | Titanium dioxide | 87.6 | 6.8 | 76.3 | 92.2 |

### Example 29

Experimental objective: feasibility determination and optimization of processes with different types of plasticizers.

Process conditions: the reactor model was -1#, and the volume of the continuous phase was 3 L.

Solvents of the dispersed phase: ethyl acetate, ethanol (absolute), and water, in a mass ratio of 5.4 : 3.2 : 1 (62 g in total).

Solutes of the dispersed phase: API, E100, N7, magnesium oxide, and plasticizers, in a mass ratio of 1.12 : 3 : 7.40 : 3 : 1 (7.76 g in total). The types of the plasticizers are shown in Table 29.

Continuous phase: 0.4% polyvinyl alcohol (05-88) aqueous solutions, continuous phase temperature: normal temperature, and continuous phase pH: 7.

Preparation process: the same as Example 1.

The indexes of the prepared taste-masking microspheres are shown in Table 29.

**Table 29**

| Test batch No. | Plasticizer type | Yield (%) | Content (%) | Encapsulatio n efficiency (%) | Dissolution rate after 30 min (%) |
|---|---|---|---|---|---|
| FC510-1 | Diethyl phthalate | 86.0 | 6.2 | 73.7 | 101.0 |
| FC510-2 | Tributyl citrate | 91.2 | 5.4 | 69.6 | 99.7 |
| FC510-3 | Triethyl citrate | 84.6 | 6.0 | 70.5 | 98.0 |
| FC707-3 | Polyethylene glycol 6000 | 87.2 | 6.2 | 75.4 | 100.3 |

### Example 30

Experimental objective: feasibility scope determination of processes additionally adding different proportions of fumaric acid.

Process conditions: the reactor model was -1#, and the volume of the continuous phase was 3 L.

Solvents of the dispersed phase: ethyl acetate, ethanol (absolute), and water, in a mass ratio of 5.4 : 3.2 : 1 (62 g in total).

Solutes of the dispersed phase: API, E100, N7, magnesium oxide, triethyl citrate, and titanium dioxide, in a mass ratio of 3.7 : 10 : 24.7 : 10 : 3.3 : 1 (feeding amount: 7.91 g), and fumaric acid additionally added in the proportions according to Table 30.

Continuous phase: 0.4% polyvinyl alcohol (05-88) aqueous solutions, continuous phase temperature: normal temperature, and continuous phase pH: 7.

Preparation process: the same as Example 1.

The indexes of the prepared taste-masking microspheres are shown in Table 30.

**Table 30**

| Test batch No. | Total feeding amount of solutes (g) | Percentage of fumaric acid relative to total amounts of solutes (wt%) | Yield (%) | Content (%) | Encapsulatio n efficiency (%) | Dissolution rate after 30 min (%) |
|---|---|---|---|---|---|---|
| FC627-2 | 7.92 | 0.08 | 90.2 | 5.6 | 71.7 | 100.9 |
| FC627-5 | 7.98 | 0.89 | 87.7 | 5.8 | 72.3 | 98.6 |
| FC630-2 | 8.33 | 5.06 | 79.8 | 5.6 | 66.4 | 102.8 |
| FC630-4 | 9.03 | 12.42 | 74.3 | 5.6 | 66.6 | 105.0 |

### Example 31

Experimental objective: feasibility scope determination and optimization and screening of processes with different proportions of API in solid feeds.

Process conditions: the reactor model was -1#, and the volume of the continuous phase was 3 L.

Solvents of the dispersed phase: ethyl acetate, and ethanol (absolute), in a mass ratio of 1.71 : 1 (55.6 g in total in the FC511-1, FC511-2, and FC511-3 batches, and 27.8 g in total in the FC511-7 batch), with water added in an amount as shown in Table 31.

Solutes of the dispersed phase: E100, magnesium oxide, and N7, in a mass ratio of 1 : 1 : 2.47 (in Table 27, the feeding amount of the three in the FC511-7 batch being 3.35 g, and the feeding amount of the three in the remaining batches being 6.7 g), and API added in an amount as shown in Table 27.

Continuous phase: 0.4% polyvinyl alcohol (05-88) aqueous solutions, continuous phase temperature: normal temperature, and continuous phase pH: 7.

Preparation process: the same as Example 1.

The indexes of the prepared taste-masking microspheres are shown in Table 31.

**Table 31**

| Test batch No. | Amou nt of API (g) | Total feeding amount of dispersed phase solutes (g) | Amount of water added in dispersed phase solvents (g) | Feeding percent age of API (%) | Yield (%) | Content (%) | Encaps ulation efficien cy (%) | Dissolutio n rate after 30 min (%) |
|---|---|---|---|---|---|---|---|---|
| FC511-1 | 0.1 | 6.8 | 5 | 1.5 | 65.1 | 2.0 | 88.2 | 96.8 |
| FC511-2 | 0.2 | 6.9 | 5 | 2.9 | 84.8 | 2.9 | 79.5 | 98.8 |
| FC511-3 | 0.56 | 7.26 | 5 | 7.7 | 84.0 | 6.4 | 68.2 | 98.8 |
| FC511-7 | 0.9 | 4.25 | 10 | 21.2 | 75.1 | 16.8 | 60.6 | 102.5 |

### Example 32

Experimental objective: feasibility scope determination and optimization and screening of processes with different proportions of total amounts of dispersed phase solutes relative to total amounts of dispersed phase solvents.

Process conditions: the reactor model was -1#, and the volume of the continuous phase was 3 L.

Solvents of the dispersed phase: ethyl acetate, ethanol (absolute), and water, in a mass ratio of 7.8 : 4.6 : 1 (60.2 g of solvent A in total).

Solutes of the dispersed phase: API, E100, magnesium oxide, and N7, in a mass ratio of 1 : 2.68 : 1.04 : 8.25, with the feeding amount of API shown in Table 32.

Continuous phase: 0.4% polyvinyl alcohol (05-88) aqueous solutions, continuous phase temperature: normal temperature, and continuous phase pH: 7.

Preparation process: the same as Example 1.

The indexes of the prepared taste-masking microspheres are shown in Table 32.

**Table 32**

| Test batch No. | API amount (g) | Total feeding amount of dispersed phase solutes (g) | Mass percentage of dispersed phase solutes relative to dispersed phase solvents (%) | Yield (%) | Content (%) | Encapsu lation efficienc y (%) | Dissoluti on rate after 30 min (%) |
|---|---|---|---|---|---|---|---|
| FC614-4 | 0.3 | 3.89 | 6.5 | 88.9 | 5.2 | 61.7 | 95.7 |
| FC614-5 | 0.56 | 7.26 | 12.1 | 88.8 | 6.3 | 69.6 | 97.1 |
| FC615-1 | 0.8 | 10.37 | 17.2 | 86.9 | 6.6 | 74.6 | 93.9 |
| FC615-5 | 1.0 | 12.96 | 21.5 | 76.0 | 6.4 | 62.6 | 93.3 |

### Example 33

Experimental objective: feasibility scope determination and optimization and screening of processes with different ratios of the continuous phase to the dispersed phase in the prescriptions.

Process conditions: the reactor model was -1#, and the volume of the continuous phase was 3 L.

Solvents of the dispersed phase: ethyl acetate, ethanol (absolute), and water, in a mass ratio of 7.0 : 4.1 : 1.

Solutes of the dispersed phase: API, E100, magnesium oxide, and N7, in a mass ratio of 1 : 2.68 : 1.04 : 8.25, with the total feeding amount of the dispersed phase solvents and the dispersed phase solutes shown in Table 33.

Continuous phase: 0.4% polyvinyl alcohol (05-88) aqueous solutions, continuous phase temperature: normal temperature, and continuous phase pH: 7.

Preparation process: the same as Example 1.

The indexes of the prepared taste-masking microspheres are shown in Table 33.

**Table 33**

| Test batch No. | API amoun t (g) | Total feeding amount of dispersed phase solvents (g) | Total feeding amount of dispersed phase solutes (g) | Ratio of mass of continuous phase to dispersed phase | Yield (%) | Cont ent (%) | Encaps ulation efficie ncy (%) | Dissoluti on rate after 30 min (%) |
|---|---|---|---|---|---|---|---|---|
| FC624-1 | 0.2 | 21.7 | 2.59 | 123.5 | 79.5 | 6.5 | 67.2 | 98.6 |
| FC624-3 | 0.56 | 60.7 | 7.26 | 44.1 | 87.1 | 6.5 | 73.0 | 96.5 |
| FC624-4 | 1.0 | 108.4 | 12.96 | 24.7 | 92.4 | 6.1 | 73.4 | 98.0 |
| FC624-5 | 1.68 | 182.1 | 21.77 | 14.7 | 88.2 | 5.1 | 58.2 | 91.4 |

### Example 34

Experimental objective: feasibility determination and optimization of processes by evaluation at different injection positions of a microsphere reactor.

Process conditions: the reactor model was -1#, and the volume of the continuous phase was 3 L.

Solvents of the dispersed phase: ethyl acetate, ethanol (absolute), and water, in a mass ratio of 8.8 : 3.9 : 1 (61.5 g in total).

Solutes of the dispersed phase: API, E100, N7, magnesium oxide, triethyl citrate, povidone, magnesium stearate, titanium dioxide, and talc powder, in a mass ratio of 5.6 : 15 : 46.2 : 5.8 : 5 : 2 : 2 : 1 : 1 (8.36 g in total).

Continuous phase: 0.4% polyvinyl alcohol (05-88) aqueous solutions, continuous phase temperature: room temperature, and continuous phase pH: 7.

Preparation process: the preparation process was the same as the preparation process of taste-masking microspheres in Example 1, only except that the injection positions were different, with the injection positions as shown in Table 34.

The indexes of the prepared taste-masking microspheres are shown in Table 34.

**Table 34**

| Test batch No. | Injection position | Yield (%) | Content (%) | Encapsulation efficiency (%) | Dissolution rate after 30 min (%) |
|---|---|---|---|---|---|
| FC527-3 | Bottom | 86.5 | 5.9 | 75.4 | 98.1 |
| FC527-4 | | 84.9 | 6.0 | 76.4 | 95.9 |
| FC527-6 | Middle part | 83.5 | 5.1 | 62.1 | 97.9 |
| FC527-7 | Upper part | 80.9 | 5.2 | 64.1 | 98.8 |

### Example 35

Experimental objective: feasibility determination of processes by evaluation and optimization of ethyl acetate, acetone and water.

Process conditions: the reactor model was -1#, and the volume of the continuous phase was 3 L.

Solvents of the dispersed phase: ethyl acetate, acetone, and water, in a mass ratio of 7.8 : 3.9 : 1 (57.1 g in total).

Solutes of the dispersed phase: API, EPO, N7, magnesium oxide, triethyl citrate, povidone, magnesium stearate, titanium dioxide, and talc powder, in a mass ratio of 5.6 : 15 : 37 : 15 : 5 : 2 : 2 : 1 : 1 (8.36 g in total).

Continuous phase: 0.4% polyvinyl alcohol (05-88) aqueous solutions, continuous phase temperature: low temperature (9°C), and continuous phase pH: 7.

Preparation process: with reference to the basic example, the number of the feed ports was 1 each side, for a total of 3; the feeding was carried out with stirring at 140 rpm for 18 min (peristaltic pump model: Signal 2S, feeding rotational speed: 12 rpm); and after feeding, the stirring speed was 100 rpm, and the stirring time was 30 min.

The indexes of the prepared taste-masking microspheres are shown in Table 35.

**Table 35**

| Test batch No. | Yield (%) | Content (%) | Encapsulation efficiency (%) | Dissolution rate after 30 min (%) |
|---|---|---|---|---|
| FC526-1 | 90.38 | 6.7 | 90.4 | 98.7 |
| FC526-2 | 88.76 | 6.8 | 90.1 | 99.7 |

### Example 36

Experimental objective: feasibility determination of processes by evaluation and optimization of ethyl acetate, ethanol and water.

Process conditions: the reactor model was -1#, and the volume of the continuous phase was 3 L.

Solvents of the dispersed phase: ethyl acetate, ethanol (absolute), and water, in a mass ratio of 7.8 : 3.9 : 1 (57.1 g in total).

Solutes of the dispersed phase: API, EPO, N10, magnesium oxide, and triethyl citrate, in a mass ratio of 1.12 : 3.2 : 7.2 : 3.0 : 1 (7.76 g in total).

Continuous phase: 0.4% polyvinyl alcohol (05-88) aqueous solutions, continuous phase temperature: low temperature (10°C), and continuous phase pH: 7.

Preparation process: with reference to the basic example, the number of the feed ports was 1 each side, for a total of 3; the feeding was carried out with stirring at 150 rpm for 16 min (peristaltic pump model: Signal 2S, feeding rotational speed: 10 rpm); and after feeding, the stirring speed was 100 rpm, and the stirring time was 30 min.

The indexes of the prepared taste-masking microspheres are shown in Table 36.

**Table 36**

| Test batch No. | Yield (%) | Content (%) | Encapsulatio n efficiency (%) | Dissolution rate after 30 min (%) |
|---|---|---|---|---|
| FC611-1 | 88.4 | 7.8 | 91.9 | 96.7 |
| FC611-2 | 88.0 | 7.6 | 92.8 | 95.2 |
| FC611-3 | 88.8 | 7.6 | 91.5 | 97.6 |

### Example 37

Experimental objective: feasibility determination of processes by evaluating the recovery and reuse of mixed solvents of ethyl acetate and ethanol.

Process conditions: the reactor model was -1#, and the volume of the continuous phase was 3 L. The organic solvents were recovered and reused, and in the recovered solvents, the content of ethyl acetate was 58.55%, and the content of ethanol was 30.22%.

Solvents of the dispersed phase: 60 g of recovered mixed solvents, and 2.4 g of absolute ethanol additionally added, with the mass ratio of ethyl acetate, ethanol and water being 5.3 : 3.1 : 1.

Solutes of the dispersed phase: API, E100, N7, magnesium oxide, triethyl citrate, and titanium dioxide, in a mass ratio of 3.7 : 10 : 24.7 : 10 : 3.3 : 1 (feeding amount: 7.91 g).

Continuous phase: 0.4% polyvinyl alcohol (05-88) aqueous solutions, continuous phase temperature: room temperature, and continuous phase pH: 7.

Preparation process: the same as Example 1.

The indexes of the prepared taste-masking microspheres are shown in Table 37.

**Table 37**

| Test batch No. | Yield (%) | Content (%) | Encapsulatio n efficiency (%) | Dissolution rate after 30 min (%) |
|---|---|---|---|---|
| FC627-3 | 87.5 | 5.9 | 73.3 | 98.7 |
| FC627-4 | 88.0 | 5.6 | 69.6 | 100.2 |

### Example 38

Experimental objective: feasibility determination of processes by evaluating the recovery and reuse of mixed solvents of ethyl acetate and acetone.

Process conditions: the reactor model was -1#, and the volume of the continuous phase was 3 L. The organic solvents were recovered and reused, and in the recovered solvents, the content of ethyl acetate was 58.26%, and the content of acetone was 29.40%.

Solvents of the dispersed phase: 60 g of recovered mixed solvents, and 3.0 g of acetone additionally added, with the mass ratio of ethyl acetate, acetone and water being 4.7 : 2.8 : 1.

Solutes of the dispersed phase: API, E100, N7, magnesium oxide, and triethyl citrate, in a mass ratio of 1.12 : 3 : 9.24 : 1.16 : 1 (7.76 g in total).

Continuous phase: 0.4% polyvinyl alcohol (05-88) aqueous solutions, continuous phase temperature: room temperature, and continuous phase pH: 7.

Preparation process: the same as Example 1.

The indexes of the prepared taste-masking microspheres are shown in Table 38.

**Table 38**

| Test batch No. | Yield (%) | Content (%) | Encapsulatio n efficiency (%) | Dissolution rate after 30 min (%) |
|---|---|---|---|---|
| FC617-1 | 83.7 | 6.3 | 73.5 | 97.8 |
| FC617-2 | 84.4 | 5.8 | 66.3 | 93.9 |

### Example 39

Experimental objective: evaluation of 2# reactor and feasibility determination of the process.

Process conditions: reactor model -2#, 6 L continuous phase.

Solvents of the dispersed phase: ethyl acetate, ethanol (absolute), and water, in a mass ratio of 9.8 : 3.8 : 1 (99.6 g in total).

Solutes of the dispersed phase: API, E100, N7, magnesium oxide, triethyl citrate, povidone, magnesium stearate, titanium dioxide, and talc powder, in a mass ratio of 5.6 : 15 : 46.2 : 5.8 : 3.3 : 2 : 2 : 1 : 1 (12.29 g in total).

Continuous phase: 0.4% polyvinyl alcohol (05-88) aqueous solutions, continuous phase temperature: low temperature (9°C), and continuous phase pH: 7.

Injection parameters: there were 2 injection points on each side, for a total of 6 injection points, and 1 discharge port in the middle part of one side. For the injection mode, a syringe with upper and lower bores (FIG. 8) was equipped; and the continuous phase and the dispersed phase were fed simultaneously, with the continuous phase fed at a rotational speed of 65 rpm (peristaltic pump model: Signal BS100-1A), and the dispersed phase fed at a rotational speed of 15 rpm (peristaltic pump model: Signal 2S), with an injection duration of 17 minutes.

The indexes of the prepared taste-masking microspheres are shown in Table 39.

**Table 39**

| Test batch No. | Yield (%) | Content (%) | Encapsulation efficiency (%) | Dissolution rate after 30 min (%) |
|---|---|---|---|---|
| FC602-1 | 88.5 | 6.7 | 86.1 | 99.2 |
| FC602-2 | 89.3 | 6.7 | 84.2 | 97.9 |
| FC606-1 | 93.4 | 6.0 | 81.0 | 99.6 |

### Example 40

Experimental objective: feasibility study and experimental validation of the preparation of taste-masking microspheres from various API drug substances such as berberine hydrochloride.

Process conditions: the reactor model was -1#, and the volume of the continuous phase was 3 L.

Solvents of the dispersed phase: ethyl acetate, ethanol, and water, in a mass ratio of 7.8 : 3.9 : 1 (57.1 g in total).

Solutes of the dispersed phase: API, EPO, N10, magnesium oxide, and triethyl citrate, in a mass ratio of 1.12 : 3 : 9.24 : 1.16 : 1 (7.76 g in total);

specific APIs were shown in Table 40.

Continuous phase: 0.4% polyvinyl alcohol (05-88), continuous phase temperature: low temperature (9°C), and continuous phase pH: 7.

Preparation process: the same as Example 1.

The evaluation of the effects of the taste-masking microspheres prepared from various APIs is shown in Table 40.

**Table 40**

| Test batch No. | API name | Conte nt of micros pheres * (%) | In vitro release rate of microsp heres * (%) | Bitterne ss (off-flavor) level of API | Bitterness (off-flavor) level of API microspher es | Percent reduction in mouth bitterness/ off-flavor level (%) | Gritty feeling: yes/no |
|---|---|---|---|---|---|---|---|
| HB722-1 | Berberine hydrochloride | 5.69 | 0.13 | 4 | 0.33 | 91.7 | No |
| XS712-1 | Sildenafil citrate | 6.88 | 0.01 | 3.33 | 0 | 100 | No |
| AA718-1 | Azithromycin | 7.3 | 0 | 4 | 0 | 100 | No |
| TC(1)718-1 | Cefuroxime axetil | 4.44 | 0.13 | 4 | 0.17 | 95.8 | No |
| FV715-1 | Vardenafil hydrochloride | 5.75 | 0.04 | 4 | 0.17 | 95.8 | No |
| EM711-2 | Metformin hydrochloride | 3.72 | 0.31 | 4 | 0.33 | 91.7 | No |
| XS(1)721-1 | Sitagliptin hydrochloride | 5.91 | 0.12 | 4 | 0.33 | 91.7 | No |
| SS713-2 | Sertraline hydrochloride | 7.18 | 0.01 | 3.83 | 0 | 100 | No |
| XP719-1 | Pyridostigmin e bromide | 3.2 | 0.50 | 3.67 | 0.50 | 86.4 | No |
| LL715-1 | Lidocaine hydrochloride | 5.26 | 0.02 | 3.17 | 0.17 | 94.7 | No |
| FF715-1 | Famotidine | 3.75 | 0.01 | 3.50 | 0 | 100 | No |
| BI718-1 | Ibuprofen | 5.68 | 0.02 | 2.83 | 0.17 | 94.1 | No |
| DA721-1 | Allitride | 3.04 | 0.01 | 4 | 0.17 | 95.8 | No |
| BB721-1 | Bepotastine besilate | 7.14 | 0.07 | 3.5 | 0.17 | 95.2 | No |
| KR722-1 | Racecadotril | 7.36 | 0.01 | 3.5 | 0.17 | 95.2 | No |
| FF(1)725-1 | Fluoxetine hydrochloride | 6.02 | 0.05 | 3.83 | 0.33 | 90.5 | No |
| FF(2)725-1 | Flucloxacillin sodium | 5.44 | 0.09 | 4 | 0.33 | 91.7 | No |
| KC725-1 | Clarithromyci n | 7.16 | 0.02 | 4 | 0.17 | 95.8 | No |
| DD(1)725-1 | Donepezil hydrochloride | 6.76 | 0.02 | 3.83 | 0.17 | 95.6 | No |
| LR722-1 | Risperidone | 6.04 | 0.05 | 2.5 | 0.33 | 86.7 | No |
| DT1206 | Domperidone | 6.74 | 0.01 | 3.83 | 0.17 | 95.7 | No |
| LB1222 | Rivaroxaban | 6.53 | 0 | 3.33 | 0 | 100 | No |
| LA1220 | Linezolid | 2.16 | 0.13 | 3.50 | 0.17 | 95.2 | No |
| TL1207 | Ticagrelor | 4.02 | 0 | 3.33 | 0 | 100 | No |
| BK1208 | Piroxicam | 5.79 | 0.36 | 3.67 | 0.33 | 90.9 | No |
| ET1221 | Irbesartan | 4.58 | 0.26 | 3.83 | 0.33 | 91.3 | No |
| AT1207 | Apremilast | 6.20 | 0 | 3.50 | 0 | 100 | No |
| LT1207 | Roflumilast | 6.80 | 0.17 | 3.67 | 0.17 | 95.5 | No |
| PN1208 | Pazopanib | 4.85 | 0 | 3.50 | 0 | 100 | No |
| LT1221 | Linagliptin | 3.88 | 0.45 | 3.67 | 0.33 | 90.9 | No |
| AQ1206 | Ondansetron | 5.13 | 0.48 | 3.83 | 0.5 | 87.0 | No |
| AL1220 | Amisulpride | 3.15 | 0.41 | 3.50 | 0.33 | 90.5 | No |
| TL1221 | Tulobuterol | 5.26 | 0.34 | 3.50 | 0.5 | 85.7 | No |
| AT1220 | Aprepitant | 5.28 | 0 | 3.67 | 0 | 100 | No |
| FA1208 | Flutamide | 5.58 | 0 | 3.67 | 0 | 100 | No |
| ML1207 | Mirabegron | 4.74 | 0.25 | 4.00 | 0.33 | 91.7 | No |
| SL1220 | Sulpiride | 3.24 | 0.43 | 3.17 | 0.33 | 89.5 | No |
| PS1221 | Pramipexole | 2.67 | 0.40 | 3.50 | 0.33 | 90.5 | No |
| DZ1222 | Dabigatran etexilate | 29.19 | 0 | 3.33 | 0 | 100 | No |
| BM1208 | Diphenhydra mine | 4.73 | 0.46 | 2.83 | 0.33 | 88.2 | No |
| MM1206 | Fluvoxamine maleate | 2.10 | 0.36 | 3.50 | 0.33 | 90.5 | No |

| | | | | | | | |
|---|---|---|---|---|---|---|---|
| Note: the test methods of the content and in vitro release rate of the taste-masking microspheres prepared from other APIs in Example 40 are the same as those of the taste-masking microspheres of vonoprazan fumarate. Note: in vitro API release rate (pH = 6.8) refers to the percentage of API dissolved during in vitro preparation relative to the total amount of API in the formulation prescription when simulating preparing dry suspensions, granules, etc., into suspension solutions or suspension granules by adding water (neutral water) before administration. | | | | | | | |

Medicaments, such as cefcapene pivoxil, cefetamet pivoxil, meropenem, moxifloxacin, linezolid, fluvoxamine maleate, rebamipide, cefteram pivoxil, verapamil, quetiapine, amisulpride, sulpiride, metoprolol, pravastatin, atomoxetine, escitalopram, tilidine hydrochloride, ondansetron, vortioxetine hydrobromide, domperidone, zopiclone, roxatidine, loperamide, diphenhydramine, epinastine, mirabegron, solifenacin, irbesartan, vortioxetine hydrobromide, ticagrelor, captopril, colesevelam hydrochloride, rasagiline, nintedanib esylate, enalapril maleate, apremilast, piroxicam, flutamide, varenicline tartrate, pazopanib, pramipexole, ripretinib, tamsulosin hydrochloride, linagliptin, micafungin, aprepitant, tigecycline, roflumilast, tenapanor hydrochloride, tulobuterol, tramadol hydrochloride, acetaminophen, colchicine, rivaroxaban, lacosamide, teneligliptin, dabigatran etexilate and odevixibat, have an API content of microspheres reaching 3% or more, an in vitro release rate of less than 0.5%, a percent reduction in mouth bitterness/off-flavor level of greater than 85%, and no gritty feeling.

### Test example 1

### Test of in vitro release rate of taste-masking microspheres

Test method for taste-masking microspheres of vonoprazan fumarate:
Determination method: the experiment was carried out according to the high-performance liquid chromatography (Pharmacopoeia of the People's Republic of China (edition 2020), Volume IV, section 0512). Chromatographic conditions: C18, 4.6 mm × 150 mm, 5 µm; mobile phase: 0.02 M dipotassium phosphate buffer (pH 5.0) - acetonitrile (65 : 35); detection wavelength: 230 nm; and column temperature: 35°C.

Sample solution for in vitro release rate test: precisely weighing 400 mg of the taste-masking microspheres of vonoprazan fumarate (containing about 20 mg of vonoprazan) prepared in the examples and placing same in a beaker, adding 20 mL of water (pH 6.8), stirring same for 30 seconds and leaving same to stand for 30 min for sampling; filtering the solution using a 0.45 µm Nylon membrane filter and a 0.22 µm Nylon membrane filter, respectively, discarding 2 mL of filtrate, and taking the subsequent filtrate.

Sample solution for content test: 10 mg of the taste-masking microspheres of vonoprazan fumarate (containing about 0.5 mg of vonoprazan) prepared in the examples were precisely weighed and placed in a 10 mL measuring flask, 1 mL of acetonitrile was added, and the resulting solution was subjected to ultrasonic treatment for 3 minutes, diluted with a diluent to mark, shaken until uniform, and filtered using a 0.45 µm Nylon membrane filter, and with 2 mL discarded, the subsequent filtrate was taken.

The results of the taste-masking microspheres of vonoprazan fumarate are shown in Table 41.

**Table 41**

| Microspher e product batch No. | API content (%) | In vitro API release rate (%) | Microspher e product batch No. | API content (%) | In vitro API release (%) |
|---|---|---|---|---|---|
| FC318-2 | 6.7 | 0.15 | FC527-7 | 5.2 | 0.11 |
| FC318-3 | 7.2 | 0.12 | FC602-2 | 6.7 | 0.10 |
| FC319-1 | 7.1 | 0.14 | FC608-3 | 6.0 | 0.09 |
| FC319-2 | 7.4 | 0.12 | FC611-1 | 7.8 | 0.11 |
| FC321-2 | 7.3 | 0.15 | FC611-2 | 7.6 | 0.11 |
| FC321-3 | 7.7 | 0.16 | FC611-3 | 7.6 | 0.12 |
| FC322-1 | 6.1 | 0.22 | FC616-4 | 4.9 | 0.15 |
| FC323-4 | 5.9 | 0.13 | FC620-2 | 4.4 | 0.16 |
| FC324-1 | 6.0 | 0.14 | FC627-5 | 5.8 | 0.10 |
| FC324-4 | 7.0 | 0.11 | FC712-2 | 4.6 | 0.10 |
| FC325-1 | 6.3 | 0.07 | FC726-1 | 5.4 | 0.25 |
| FC326-4 | 7.0 | 0.08 | FC726-2 | 5.5 | 0.18 |
| FC326-5 | 8.5 | 0.07 | FC726-3 | 5.9 | 0.20 |
| FC421-2 | 6.0 | 0.15 | FC726-4 | 5.7 | 0.22 |
| FC421-5 | 5.8 | 0.12 | FCC328-1 | 8.4 | 0.32 |
| FC422-10 | 5.0 | 0.11 | FCC328-2 | 8.0 | 0.46 |
| FC424-3 | 6.5 | 0.30 | FCC328-3 | 9.3 | 0.43 |
| FC424-7 | 5.9 | 0.11 | FCC328-4 | 7.6 | 0.50 |
| FC424-8 | 6.4 | 0.14 | FCC329-1 | 7.2 | 0.37 |
| FC426-6 | 7.1 | 0.15 | FCC329-2 | 7.8 | 0.47 |
| FC427-2 | 6.5 | 0.14 | FCC329-3 | 7.3 | 0.37 |
| FC428-2 | 5.5 | 0.08 | FCC329-4 | 8.7 | 0.46 |
| FC428-5 | 6.0 | 0.16 | FCC330-1 | 9.7 | 0.34 |
| FC428-8 | 5.8 | 0.09 | FCC330-2 | 9.0 | 0.48 |
| FC429-1 | 5.7 | 0.12 | FCC330-3 | 9.7 | 0.41 |
| FC429-4 | 5.4 | 0.11 | FCC330-4 | 9.6 | 0.42 |
| FC509-2 | 6.6 | 0.09 | FCC406-1 | 8.8 | 0.45 |
| FC526-1 | 6.8 | 0.09 | FCC406-2 | 9.7 | 0.50 |
| FC526-2 | 6.8 | 0.09 | FCC406-3 | 9.2 | 0.47 |
| FC527-4 | 5.9 | 0.11 | FCC406-4 | 10.4 | 0.39 |
| FC527-6 | 5.1 | 0.16 | -- | -- | -- |

The taste-masking microspheres prepared from vonoprazan fumarate in examples of the present disclosure all have an in vitro release rate of 0.5% or less.

Note: The FCC batch number represents examples of preparing microspheres using a pH-independent carrier, and the preparation method for the in vitro release rate test is as follows: precisely weighing 400 mg of the taste-masking microspheres of vonoprazan fumarate (containing about 20 mg of vonoprazan) prepared in the examples and placing same in a beaker, adding 20 mL of water (pH 6.8), stirring same for 30 seconds and leaving same to stand for 3 min for sampling; filtering the solution using a 0.45 µm Nylon membrane filter and a 0.22 µm Nylon membrane filter, respectively, discarding 2 mL of filtrate, and taking the subsequent filtrate.

### Test example 2

### Test of taste-masking effect of taste-masking microspheres

### 1. Taste test

The taste-masking test methods of the taste-masking microspheres prepared from various APIs in the present disclosure are the same. The following is a specific description with the taste-masking microspheres of vonoprazan fumarate as an example.

### (1) Preparation of bitterness standard solution

About 0.25 g of a drug substance (e.g., vonoprazan) was prepared into a 0.1% aqueous solution of vonoprazan, thus obtaining a bitterness-4 standard solution. The above solution was additionally diluted 100, 20, 10, and 5 times, as bitterness-0, bitterness-1, bitterness-2, and bitterness-3 standard solutions, respectively. The bitterness criteria are shown in Table 42.

**Table 42**

| Bitterness criteria | Bitterness standard solution (vonoprazan concentration) |
|---|---|
| Bitterness 4 | Intense bitterness (0.1%) |
| Bitterness 3 | Significant bitterness (0.02%) |
| Bitterness 2 | Acceptable bitterness (0.01%) |
| Bitterness 1 | Mild bitterness (0.005%) |
| Bitterness 0 | No bitterness (0.001%) |

### (2) Preparation of sample standard solution

Vonoprazan microsphere products from four batches were weighed and prepared into 1 mg/mL (0.1%) vonoprazan-containing suspensions, which were stirred for 30 s and left to stand for 30 min for taste samples use. The samples were numbered, as specifically shown in Table 43.

**Table 43**

| | | | | |
|---|---|---|---|---|
| Test product batch No. | FC611-1 | FC611-2 | FC611-3 | FC509-2 |
| Sample No. | No. I | No. II | No. III | No. IV |
| Test product batch No. | FCC328-1 | FCC328-4 | FCC329-2 | - |
| Sample No. | No. V | No. VI | No. VII | - |

### (3) Taste test scheme

12 healthy volunteers (6 males and 6 females) aged 22-35 were selected, with their mouths rinsed 3 times with water before tasting. Each taster first attempted purified water and then bitterness standard solutions 0, 1, 2, 3, and 4 in sequence to establish an individual differential bitter taste standard. The standard solution for each attempt was 1 mL, and the bitterness standard solution was dripped onto the center of the tongue and left in the mouth for about 30 s. Each sample attempt should be at least 15 minutes apart.

The samples were tested, with each subject tasting the samples according to the individualized standard and method established by the bitterness standard solution. The levels of bitterness were recorded.

### (4) Bitterness experiment results

### 1) Bitterness experiment results of standard solutions

The bitterness experiment results of the standard solutions are shown in Table 44.

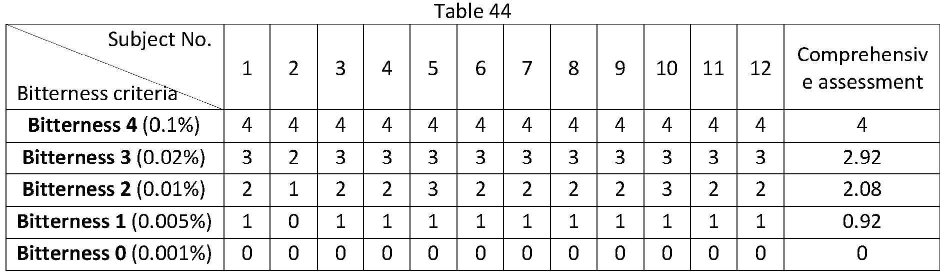

### 2) Bitterness experiment results of sample solutions

The experimental results of the taste-masking microsphere taste samples prepared in the examples are shown in Table 45.

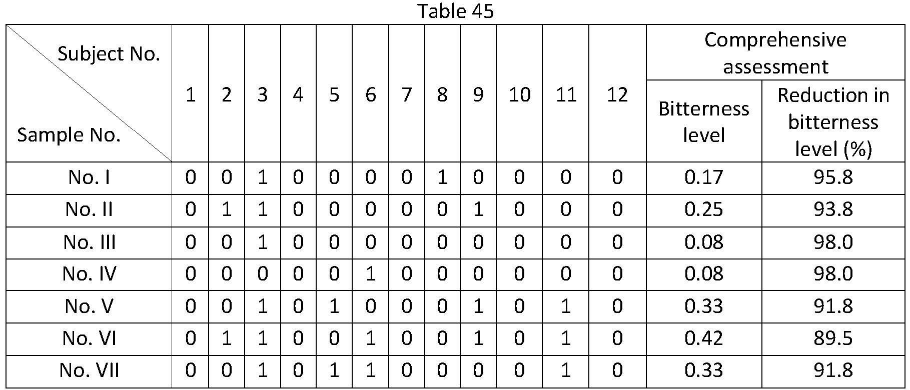

3) Test results of gritty feeling of sample solutions: It is recorded as 1 when there is a gritty feeling and 0 without a gritty feeling. The results are shown in Table 46.

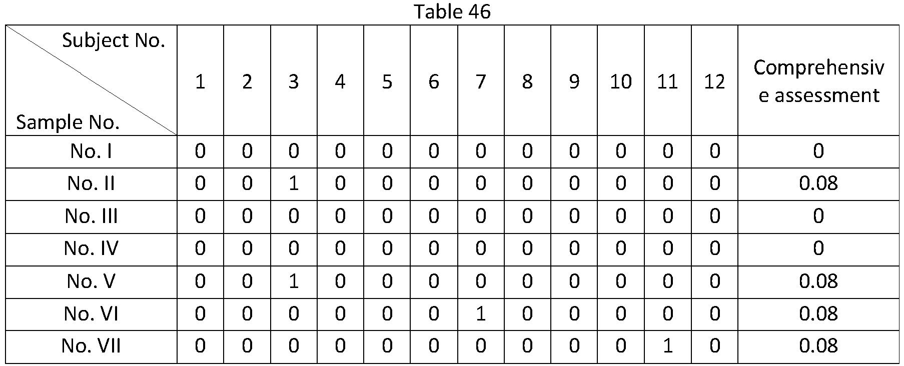

The mouthfeel effects of the taste-masking microspheres prepared from various APIs in the examples were tested in the present disclosure. The results show that all taste-masking microspheres have a significant effect in reducing bitterness, with a bitterness reduction level of 85% or more, and have no gritty feeling.

### 2. Electronic tongue detection test

(1) Instrument model: An electronic tongue model SA402B from Insent, Japan, was used, which is a lipid membrane-based potential type electronic tongue. The lipid membrane consists of a variety of potential electrodes, which is similar to the lipid membrane of the cell wall of human taste bud cells and can perceive and distinguish the taste of different sample solutions.
(2) Bitterness analysis system sensor and performance description:
AN0: base salt-like bitterness (B-bitterness 2)
BT0: alkaline bitterness (H-bitterness)

(3) Sample preparation method:
API solution: About 27 mg of an API product was weighed, placed in a 40 mL measuring flask, dissolved with water to mark and shaken until uniform.
Taste-masking microsphere solution: About 400 mg of the taste-masking microspheres (batch No.: FC509-2) were weighed and placed in a 40 mL measuring flask; water was added to mark; and the solution was shaken for 30 s, left to stand for 10 minutes and filtered with a filter paper, and the filtrate was collected as a sample solution.
The concentration of the API solution and the taste-masking microsphere solution is about 0.5 mg/mL, calculated based on vonoprazan.

(4) Test method
Equilibration: the sensor was firstly cleaned in a cleaning solution for 90 s and then cleaned with a reference solution for 120 s and then another reference solution for 120 s, and then the sensor is zeroed at the equilibrium position for 30 s.
Test: 35 mL of a sample was placed in a specific sample cup of an electronic tongue for testing for 30 s, and the test value was output; then the sensor was cleaned with a reference solution for 3 s and then inserted in a new reference solution for 30 s. The test was repeated four times, with the first test value removed, the data of the last three times were averaged as the test result.

(5) The test results are shown in Table 47:

**Table 47**

| Sample | AN0 | | | Average | BT0 | | | Average |
|---|---|---|---|---|---|---|---|---|
| | B-bitterness 2 | | | | H-bitterness | | | |
| API | 67.32 | 66.04 | 64.49 | 65.95 | 1.66 | 1.73 | 1.7 | 1.7 |
| FC509-2 | 9.02 | 9.97 | 10.44 | 9.81 | -0.43 | -0.47 | -0.61 | -0.5 |
| Reduction in bitterness after taste masking (%) | 85.13 | | | | 100 | | | |

| | | | | | | | | |
|---|---|---|---|---|---|---|---|---|
| Note: the reference is 0, and a detection value > 0 means bitter, and < 0 means no detectable bitter taste. | | | | | | | | |

(6) The radar chart obtained from the test is shown in FIG. 9, where k1 represents API, and k2 represents taste-masking microspheres of vonoprazan fumarate F509-2.

### Test example 3

### Relevant substance test of taste-masking microspheres of vonoprazan fumarate

Detection methods: an octadecylsilane chemically bonded silica was used as a filler; 0.025 mol/L dipotassium phosphate solution (with pH adjusted to 6.0 with phosphoric acid) - acetonitrile (80 : 20) was used as mobile phase A, and 0.025 mol/L dipotassium phosphate solution (with pH adjusted to 6.0 with phosphoric acid) - acetonitrile (30 : 70) was used as mobile phase B, and linear gradient elution was carried out according to Table 48; column temperature: 30°C; flow rate: 1.0 mL per minute; detection wavelength: 230 nm; and injection volume: 10 µL.

**Table 48**

| Time (min) | Mobile phase A (%) | Mobile phase B (%) |
|---|---|---|
| 0 | 100 | 0 |
| 18 | 74 | 26 |
| 47 | 0 | 100 |
| 50 | 0 | 100 |
| 50.1 | 100 | 0 |
| 60 | 100 | 0 |

The results are as shown in Table 49. The impurities G and F are both around 0.1%, the sum of impurities is less than 0.4%, the number of impurities is 7, and the purity of the liquid phase is more than 99.6%. The mass of the relevant substances of microspheres meets the standard requirements of the formulation.

**Table 49**

| Name | FC527-3 | | FC527-4 | | FC606-1 | |
|---|---|---|---|---|---|---|
| | Retention time (min) | Content (%) | Retention time (min) | Content (%) | Retentio n time (min) | Content (%) |
| Vonoprazan | 11.683 | 99.64 | 11.680 | 99.61 | 12.067 | 99.45 |
| G | 7.701 | 0.10 | 7.696 | 0.11 | 8.062 | 0.11 |
| Maximum individual impurity | 25.447 | 0.10 | 25.453 | 0.10 | 25.843 | 0.14 |
| Total impurity (%) | 0.36 | | 0.39 | | 0.55 | |

| Name | FCC328-1 | | FCC328-4 | | FCC329-2 | |
|---|---|---|---|---|---|---|
| | Retention time (min) | Content (%) | Retention time (min) | Content (%) | Retentio n time (min) | Content (%) |
| Vonoprazan | 11.537 | 99.83 | 11.563 | 99.83 | 11.564 | 99.75 |
| G | 7.794 | 0.09 | 7.823 | 0.08 | 7.829 | 0.17 |
| Maximum individual impurity | 13.747 | 0.04 | 13.763 | 0.04 | 13.778 | 0.04 |
| Total impurity (%) | 0.17 | | 0.18 | | 0.25 | |

### Test example 4

The vonoprazan fumarate microspheres (FC606-1) were subjected to a particle size distribution test, and the result was shown in Table 50. Dv(10) is 159 µm, Dv(50) is 249 µm, and Dv(90) is 379 µm. "Dv(50) is 249 µm", that is, 50% of the microspheres have a particle size of 250 µm or less.

**Table 50**

| Batch No. | Particle size test | | |
|---|---|---|---|
| | Test results/µm | Average/µm | Test method |
| FC606-1 | Dv(10) | 159.3 | Light scattering method (dry method)* |
| | Dv(50) | 248.7 | |
| | Dv(90) | 378.7 | |

| | | | |
|---|---|---|---|
| *: the third method - light scattering method in Pharmacopoeia of the People's Republic of China (edition 2020), Volume IV, section 0982 | | | |

### Test example 5

Observation of appearance and morphology of taste-masking microspheres

The appearance and morphology of microspheres of vonoprazan fumarate were observed using a scanning electron microscope. The results are shown in FIG. 10 and FIG. 11. As shown in FIG. 10, the microspheres of vonoprazan fumarate have a complete spherical shape, a smooth and non-porous surface morphology, good surface smooth finish, and high roundness. As shown in FIG. 11, the microspheres contain a large number of nanoscale pores inside and are uniformly sized (without a coating layer). As shown in FIG. 10, the microspheres have a particle size in a range of 50-280 µm, with uniform particle size and complete spherical shape.

The microspheres of vonoprazan fumarate (batch No. FC606-1) prepared by the present disclosure were observed using a microscope. The result is shown in FIG. 12.

### Test example 6

### Dissolution test of microspheres of vonoprazan fumarate

Dissolution scheme: The taste-masking microspheres prepared in the examples were subjected to a dissolution test according to the provisions in Pharmacopoeia of the People's Republic of China (edition 2020), Volume IV, section 0931, Determination Methods for Dissolution Rate and Release Rate, Method 2. The dissolution methods are as follows:

Dissolution medium: pH 1.0, a 900 mL buffer solution, rotational speed: 50 rpm.

Determination method: high-performance liquid chromatography. The results are shown in Table 51.

**Table 51**

| Batch No. | 5 min (%) | 10 min (%) | 15 min (%) | 30 min (%) | 45 min (%) | 60 min (%) |
|---|---|---|---|---|---|---|
| FC611-1 | 36.7 | 57.8 | 76.2 | 96.4 | 99.1 | 99.9 |
| FC611-2 | 31.9 | 56.8 | 74.6 | 97.1 | 99.2 | 100.1 |
| FC611-3 | 45.1 | 70.9 | 87.1 | 99.3 | 100.4 | 100.7 |
| FCC328-1 | 59.0 | 67.6 | 77.9 | 92.0 | 93.5 | 94.8 |
| FCC328-4 | 59.4 | 78.9 | 91.1 | 92.0 | 93.6 | 94.4 |
| FCC329-2 | 60.3 | 88.0 | 89.0 | 92.1 | 94.7 | 94.1 |

### Test example 7

### Density and fluidity test of microspheres of vonoprazan fumarate

The micromeritics determination results of microspheres of vonoprazan fumarate of batch No. FC527-3 are shown in Table 52.

**Table 52**

| Number of measurements of sample | Bulk density (g/mL) | Tap density (g/mL) | Carr index | Hausner ratio |
|---|---|---|---|---|
| No.1 | 0.306 | 0.352 | 17.12 | 1.17 |
| No.2 | 0.297 | 0.353 | | |
| No.3 | 0.301 | 0.353 | | |

### Test example 8

### Crystallinity test of microspheres of vonoprazan fumarate

The vonoprazan fumarate-containing microspheres and blank microspheres with the same auxiliary materials were tested by XRD (X-ray diffraction), specifically as shown in Table 53. As analyzed from the detection data in Table 53, it can be seen that the deviation of the diffraction peaks at four positions is within ± 0.2°, so the measured crystallinity of the vonoprazan fumarate-containing microspheres of the present disclosure is mainly contributed by the auxiliary materials in the blank microspheres, that is, the vonoprazan fumarate in the medicament-containing microspheres has almost no crystallinity, indicating that the vonoprazan fumarate in the medicament-containing microspheres exists in the form of molecular dispersion rather than crystalline dispersion.

**Table 53**

| Sample name | Sample batch No. | Degree of crystallinity (%) | Diffraction angle 2θ (°) |
|---|---|---|---|
| Blank microspheres | FC606-1* | 44.5 | 25.375, 36.988, 37.894, 42.968 |
| Microspheres of vonoprazan fumarate | FC606-1 | 52.9 | 25.277, 36.890, 37.808, 42.857 |

The medicament of vonoprazan fumarate exists inside the taste-masking microspheres in the form of molecular dispersion in an amorphous form, which is beneficial to the dissolution of the medicament in the body, because the release of medicaments from the microspheres first occurs in the amorphous region and then in the crystalline region. Therefore, a medicament existing in microspheres as molecular dispersion is more favorable for medicament delivery and release than as particulate dispersion.

### Application example 1

Preparation of compound formulation in the dosage form of dry suspension of vonoprazan fumarate (microsphere form), amoxicillin and rifabutin

### (1) Preparation method of amoxicillin granules (1.0 g) in compound formulation

The preparation procedures of the amoxicillin granules were: pre-treatment of raw/auxiliary materials, pre-mixing, soft material preparation, extrusion, spheronization, drying, and granulation. The amoxicillin granules consisted of the prescriptions shown in Table 54:

**Table 54**

| Name of component | Function | Unit weight percentage | Amount in single formulation (g) |
|---|---|---|---|
| Amoxicillin | Active ingredient | 41.7% | 2.40 |
| Microcrystalline cellulose PH101 | Filler | 23.6% | |
| Sorbitol | Filler | 33.38% | |
| Silica | Glidant | 1.04% | |
| Edible sorghum red pigment | Colorant | 0.08% | |
| Xanthan gum | Binder | 0.2% | |
| 80% ethanol solution | Wetting agent | Q.S. (quantum sufficit) | |
| Total weight per 1000 formulation units (2.4 kg) | | | |

The test results of focused items of amoxicillin granules are shown in Table 55:

**Table 55**

| Intermediate batch No. | Content | Particle size | | Moisture |
|---|---|---|---|---|
| | 95% - 105% of labeled amount | 30 mesh: all should pass through | Passing through 120 mesh: not more than 15% | Should be less than 6.0% |
| A611-1 | 98.5% | 0% | 10% | 5.6% |
| A611-2 | 99.2% | 0% | 8% | 5.3% |
| A611-3 | 98.8% | 0% | 12% | 5.1% |

### (2) Preparation method of rifabutin granules (labeled amount: 50 mg, 75 mg, 100 mg, 125 mg, and 150 mg) in compound formulation

The preparation procedures of the rifabutin granules were: pre-treatment of raw/auxiliary materials, pre-mixing, extrusion, spheronization, drying, and granulation. The various labeled amounts of rifabutin granules consisted of the prescriptions shown in Table 56:

**Table 56**

| Function | Active ingredient | Filler | | Solubilizer | Glidant |
|---|---|---|---|---|---|
| Name of component | Rifabutin (%) | Microcrystallin e cellulose (%) | Sorbitol (%) | Sodium dodecyl sulfate (%) | Silica (%) |
| L611-2 (50 mg) | 3.13 | 18.04 | 77.88 | 0.85 | 0.10 |
| L610-1 (75 mg) | 4.69 | 16.13 | 77.88 | 1.20 | 0.10 |
| L611-3 (100 mg) | 6.25 | 10.15 | 82.00 | 1.50 | 0.10 |
| L611-4 (125 mg) | 7.81 | 8.09 | 82.00 | 2.00 | 0.10 |
| L611-5 (150 mg) | 9.38 | 5.52 | 82.00 | 3.00 | 0.10 |

| | | | | | |
|---|---|---|---|---|---|
| Note: # represents that the total weight of 1000 formulation units is 1600 g | | | | | |

The results of the test items of rifabutin granules are shown in Table 57:

**Table 57**

| Intermediate batch No. | Content | Particle size | | Moisture |
|---|---|---|---|---|
| | 95% - 105% of labeled amount | 30 mesh: all should pass through | Passing through 120 mesh: not more than 10% | Should be less than 1.0% |
| L611-2 (50 mg) | 98.7% | 0% | 8% | 0.51% |
| L610-1 (75 mg) | 99.6% | 0% | 6% | 0.53% |
| L611-3 (100 mg) | 99.1% | 0% | 3% | 0.61% |
| L611-4 (125 mg) | 99.5% | 0% | 5% | 0.57% |
| L611-5 (150 mg) | 98.8% | 0% | 4% | 0.53% |

(3) Preparation of compound formulation in the dosage form of dry suspension of microspheres of vonoprazan fumarate, amoxicillin and rifabutin. The prescription composition of the compound formulation is shown in Table 58:

**Table 58**

| Material name | Batch No. | Specificati ons (mg) | Content (%) | Percentage of ingredient in compound formulation (%) | Amount in single formulation (g) |
|---|---|---|---|---|---|
| Rifabutin granules | L611-2 | 50 | 3.13 | 33.26 | 1.6 |
| | | 75 | 4.69 | | |
| | | 100 | 6.25 | | |
| | | 125 | 7.81 | | |
| | | 150 | 9.38 | | |
| | HFC613-2* | 20 | 6.8 | 8.32 | 0.4 |
| | A611 | 1000 | 41.7 | 49.90 | 2.4 |
| Xanthan gum | Suspending agent | | | 3.18 | 0.41 |
| Sodium saccharin | Sweetener | | | 0.10 | |
| Sodium cyclamate | | | | | |
| Magnesium stearate | Lubricant | | | 3.24 | |
| Edible strawberry powder essence | Flavor modifier | | | 1.00 | |
| Total theoretical feeding amount: 4810 g | | | | 100 | 4.81 |

| | | | | | |
|---|---|---|---|---|---|
| *Note: HFC613-2 is prepared by mixing FC407-1, FC526-1, FC526-2, FC611-1, FC611-2, FC611-3 batches, with a content after mixing being 7.3%. | | | | | |

The test data for the total blend granules of the compound formulation are shown in Table 59:

**Table 59**

| Batch No. | Specificatio n of formulation (mg) | Vonoprazan (n = 10) | | Amoxicillin (n = 10) | | Rifabutin (n = 10) | | Sedimentat ion volume ratio |
|---|---|---|---|---|---|---|---|---|
| | | Average content | RSD | Average content | RSD | Average content | RSD | |
| FAL614 -5 | 20 : 1000 : 50 | 98.5% | 2.3% | 99.1% | 2.9% | 98.7% | 3.1 | 0.93 |
| FAL614 -6 | 20 : 1000 : 75 | 99.2% | 3.1% | 99.6% | 3.2% | 98.33% | 2.5 | 0.95 |
| FAL615 -1 | 20 : 1000 : 100 | 99.6% | 3.1% | 99.4% | 2.1% | 98.8% | 2.0 | 0.97 |
| FAL615 -2 | 20 : 1000 : 125 | 98.9% | 2.6% | 99.1% | 2.3% | 99.0% | 1.8 | 0.96 |
| FAL615 -3 | 20 : 1000 : 150 | 98.9% | 2.6% | 99.4% | 1.6% | 98.9% | 2.2 | 0.97 |

The total blend granules of this compound formulation can be packaged into an oral dry suspension, with a bag size of 4.81 g, containing 20 mg of vonoprazan, 1000 mg of amoxicillin, and 50-150 mg of rifabutin. It is clinically used to eradicate Hp infection.

The test results of the compound formulation are shown in Table 60:

**Table 60**

| Batch No. | Specification of formulation (mg) | Content uniformity (A + 2.2S should not greater than 20.0) | | | Moisture (%) | Sedimentation rate (%) |
|---|---|---|---|---|---|---|
| | | F^{*} | A^{*} | L^{*} | | |
| FAL614-5 | 20 : 1000 : 50 | 16.2 | 13.1 | 17.2 | 8.2% | 0.95 |
| FAL614-6 | 20 : 1000 : 75 | 17.2 | 14.3 | 16.1 | 8.5% | 0.97 |
| FAL615-1 | 20 : 1000 : 100 | 17.6 | 13.6 | 17.1 | 8.6% | 0.93 |
| FAL615-2 | 20 : 1000 : 125 | 15.3 | 13.6 | 14.8 | 8.4% | 0.98 |
| FAL615-3 | 20 : 1000 : 150 | 15.7 | 13.9 | 15.3 | 8.1% | 0.96 |

| | | | | | | |
|---|---|---|---|---|---|---|
| *Note: F represents vonoprazan, A represents amoxicillin, and L represents rifabutin. | | | | | | |

### Application example 2

Preparation of dry suspension of vonoprazan fumarate (microspheres form)

### 1. The prescription composition is shown in Table 61.

**Table 61**

| Raw/auxiliary material | Function | Content in percentages (%) | Unit dose weight (mg) |
|---|---|---|---|
| Microspheres of vonoprazan fumarate (batch No. HFC613-2) | Active ingredient | 80.0% (based on vonoprazan) | 400 |
| Xanthan gum | Suspending agent | 10.0% | 50 |
| Citric acid anhydrous | pH regulator | 4.0% | 20 |
| Magnesium stearate | Glidant | 2.0% | 10 |
| Silica | Anti-sticking agent | 1.0% | 5 |
| Aspartame | Sweetener | 2.0% | 10 |
| Peach powder essence | Flavoring agent | 1.0% | 5 |
| Total | | 100% | 500 |
| Total theoretical feeding amount: 50 g | | | |

### 2. Preparation process

Microsphere preparation → weighing according to prescription ratio → mixing → intermediate detection → granule packing → finished product, with a specification of 10 mg or 20 mg (based on vonoprazan).

### 3. The product testing is shown in Table 62:

**Table 62**

| Batch No. | Content | Loss on drying | pH | Dissolution rate | Sedimentation volume ratio |
|---|---|---|---|---|---|
| | 95% - 105% | Should be less than 2.0% | 4.0-7.0 | 30 minutes, not less than 85% | Not less than 0.90 |
| FCP622 | 98.5% | 1.2% | 6.2 | 90% | 0.95 |

### Application example 3

Preparation of vonoprazan fumarate tablets, capsules and granules 1. The prescription composition is shown in Table 63.

**Table 63**

| Raw/auxiliary material | Function | Content in percentages (%) | Unit dose weight (mg) |
|---|---|---|---|
| Taste-masking microspheres of vonoprazan fumarate (batch No. HFC613-2) | Active ingredient | 87.91% (based on vonoprazan) | 400.0 |
| Microcrystalline cellulose | Excipient | 9.0% | 45.0 |
| Fumaric acid | Stabilizer | 1.0% | 5.0 |
| Magnesium stearate | Glidant | 2.0% | 10.0 |
| Silica | Anti-sticking agent | 0.09% | 0.45 |
| Total | | 100% | 460.45 |
| Total theoretical feeding amount: 46 g | | | |

### 2. Preparation procedures

The specification was 10 mg or 20 mg (based on vonoprazan).
Granules: microsphere preparation → weighing according to prescription ratio → mixing → intermediate detection → granule packing → finished product.
Tablets: microsphere preparation → weighing according to prescription ratio → mixing → intermediate detection → tableting → finished product.
Capsules: microsphere preparation → weighing according to prescription ratio → mixing → intermediate detection → capsule filling → finished product.

### 3. The product testing is shown in Table 64:

**Table 64**

| Finished product batch No. | Content | Moisture | Dissolution rate | Fill weight variation |
|---|---|---|---|---|
| | 95% - 105% | Should be less than 3.0% | 30 minutes, not less than 85% | ± 5% |
| FC625-1 (capsules) | 99.3% | 1.8% | 92% | +2.6% to -2.0% |
| FC625-2 (tablets) | 98.5% | 1.6% | 90% | +2.3% to -2.0% |
| FC625-3 (granules) | 99.0% | 1.3% | 95% | +2.1% to -1.5% |

### Comparative example 1

### Comparison of adding dropwise above reactor liquid surface

Process conditions: the reactor model was -1#, and the volume of the continuous phase was 3 L.

Solvents of the dispersed phase: ethyl acetate, ethanol (absolute), and water, in a mass ratio of 7.8 : 3.9 : 1 (57.1 g in total).

Solutes of the dispersed phase: API, EPO, N10, magnesium oxide, and triethyl citrate, in a mass ratio of 1.12 : 3.2 : 7.2 : 3.0 : 1 (7.76 g in total).

Continuous phase: 0.4% polyvinyl alcohol (05-88) aqueous solutions, continuous phase temperature: low temperature (10°C), and continuous phase pH: 7.

Preparation process: the injection method was adding dropwise above the reactor liquid surface, and the other processes were the same as in Example 36.

The indexes of the prepared taste-masking microspheres are shown in Table 65.

**Table 65**

| Test batch No. | Addition method | Yield (%) | Conten t (%) | Encapsul ation efficiency (%) | Loss on drying (%) | Dissolution rate after 30 min (%) |
|---|---|---|---|---|---|---|
| FC628-2 | Adding dropwise above liquid surface | 64.8 | 4.8 | 53.2 | 1.5 | 98.1 |
| FC628-3 | Adding dropwise above liquid surface | 72.5 | 4.3 | 46.4 | 2.0 | 93.2 |
| FC628-4 | Adding dropwise above liquid surface | 67.1 | 5.4 | 57.9 | 1.9 | 96.7 |

As can be seen from Table 61, film formation occurs when the dispersed phase material is added dropwise above the liquid surface, and the encapsulation efficiency is low (less than 60%).

### Comparative example 2

### Alkalizing agents with low microsphere content and encapsulation efficiency

Process conditions: the reactor model was -1#, and the volume of the continuous phase was 3 L.

Solvents of the dispersed phase: ethyl acetate, ethanol (absolute), and water, in a mass ratio of 5.4 : 3.2 : 1 (62 g in total).

Solutes of the dispersed phase: API, EPO N10, an alkalizing agent, triethyl citrate, magnesium stearate, talc powder, and titanium dioxide, in a mass ratio of 5.6 : 15 : 37 : 15 : 5 : 2 : 1 : 1 (8.16 g in total). The types of the alkalizing agent are shown in Table 66.

Continuous phase: 0.4% polyvinyl alcohol (05-88) aqueous solutions, continuous phase temperature: normal temperature, and continuous phase pH: 7.

### Preparation process: the same as Example 26.

The indexes of the prepared taste-masking microspheres are shown in Table 66.

**Table 66**

| Test batch No. | Alkalizing agent type | Yield (%) | Content (%) | Encapsulatio n efficiency (%) | Dissolution rate after 30 min (%) |
|---|---|---|---|---|---|
| FC318-1 | Magnesium aluminum silicate | 26.4 | 2.2 | 26.4 | 103.0 |
| FC319-3 | Sodium acetate | 65.6 | 2.7 | 25.3 | 103.3 |
| FC319-4 | Sodium citrate | 67.0 | 2.9 | 27.4 | 101.0 |

In Table 66, the taste-masking microspheres prepared with alkalizing agents such as sodium citrate all have a low yield and content and an encapsulation efficiency of less than 30%.

### Comparative example 3

### Carrier auxiliary materials replacing matrix-type carrier N7 or N10

Process conditions: the reactor model was -1#, and the volume of the continuous phase was 3 L.

Solvents of the dispersed phase: ethyl acetate, ethanol (absolute), and water, in a mass ratio of 5.4 : 3.2 : 1 (62 g in total).

Solutes of the dispersed phase: API, E100, celluloses (see Table 67), and magnesium oxide, in a mass ratio of 1 : 2.68 : 6.61 : 2.68 (7.26 g in total).

Continuous phase: 0.4% polyvinyl alcohol (05-88) aqueous solutions, continuous phase temperature: normal temperature, and continuous phase pH: 7.

Preparation process: the same as Example 25.

The indexes of the prepared taste-masking microspheres are shown in Table 67.

**Table 67**

| Test batch No. | Name of Cellulose series | Yield (%) | Content (%) | Encapsulatio n efficiency (%) | Dissolution rate after 30 min (%) |
|---|---|---|---|---|---|
| FC505-1 | Microcrystalline cellulose | 79.20 | 3.29 | 33.19 | 84.42 |
| FC505-2 | Low-substituted hydroxypropyl cellulose | 11.95 | 4.30 | 6.56 | --- |
| FC505-3 | Sodium carboxymethyl cellulose | 6.15 | 7.76 | 6.24 | --- |
| FC505-4 | Crospovidone | 85.40 | 3.43 | 37.31 | 93.41 |

When a matrix-type carrier N7 or N10 is replaced with cellulose such as microcrystalline cellulose and crospovidone in Table 67, the tested encapsulation efficiencies are all less than 40%.

### Comparative example 4

### Extrusion-spheronization preparation process of vonoprazan fumarate granules

### 1. Prescription composition:

**Table 68**

| Material name | Vonopraz an fumarate | EPO | N7 | Magnesi um oxide | Magnesi um stearate | Titaniu m dioxide | Silica |
|---|---|---|---|---|---|---|---|
| Function | Active ingredient | Carrie r | Carrie r | Alkalizin g agent | Lubrican t | Opacifie r | Lubrican t |
| Prescription percentage (%) | 7.12 | 19.08 | 58.78 | 7.38 | 3.18 | 1.28 | 3.18 |
| Total weight per formulation unit (g) 0.35 | | | | | | | |

2. Preparation procedures: Material mixing (direct mixing of pH-dependent auxiliary materials) - wetting - extrusion into strips - cutting into cylinders - spheronization into micropellets with a rounder - drying - obtaining extruded rounded micropellets (sticking occurred in the extrusion-spheronization processes, resulting in products in the shape of strips and agglomerates, which were then dried and crushed into granules).

### 3. Product testing:

**Table 69**

| Test item | Conte nt (%) | In vitro release rate (%) | pH 1.0 medium dissolution rate (%) | | Taste test | | | |
|---|---|---|---|---|---|---|---|---|
| | | | 30 min | 60 min | Bitterne ss level of API | Bitterness level of extruded rounded granules | Percent reduction in bitterness (%) | Gritty feeling |
| F701-2 | 5.77 | 10.46 | 90.1 | 97.2 | 4 | 2.75 | 31.25 | 0.33 |

| | | | | | | | | |
|---|---|---|---|---|---|---|---|---|
| Note: The taste test standards and schemes in Table 69 were the same as those in Test example 2. | | | | | | | | |

### 2. Other test data:

### (1) Particle size distribution

**Table 70**

| Batch No. | Particle size test | | |
|---|---|---|---|
| | Test results/µm | Average/µm | Test method |
| F701-2 | Dv(10) | 84.6 | Dry particle size |
| | Dv(50) | 253.0 | |
| | Dv(90) | 468.0 | |

### (2) Scanning electron microscope

The extruded rounded granules have a scanning electron microscope appearance image as shown in FIG. 13, and a section image as shown in FIG. 14.

The vonoprazan fumarate granules prepared by using an extrusion-spheronization process show a not-rounded appearance, large particle size, a Dv(90) reaching 468 µm, a reduction in bitterness in a taste test by 31.25%, a gritty feeling of 0.33, and a poor taste-masking effect.

### Comparative example 5

### Wet granulation-fluidized coating preparation process

### 1. Prescription composition

**Table 71**

| Material name | Fumaric acid Vonopraza n | Sorbitol | Microcry stalline cellulose | Hydroxyp ropyl cellulose | Croscarmello se sodium | Opadry amb II |
|---|---|---|---|---|---|---|
| Function | Active ingredient | Excipien t | Excipient | Binder | Disintegratin g agent | Coating layer |
| Prescription percentage (%) | 6.19 | 71.13 | 9.26 | 1.39 | 4.63 | 7.41 |
| Total weight per formulation unit (g) 0.35 | | | | | | |

### 2. Preparation procedures: Material mixing - wetting - granulation - drying - fluidized coating - coated granules.

The technical specifications of the coated granules are shown in Table 72.

**Table 72**

| | |
|---|---|
| Material coating weight gain (%) | 11.0% |
| Yield (%) | 91.1% |

### 3. Product testing

**Table 73**

| Test item | Cont ent (%) | In vitro release rate (%) | pH 1.0 medium dissolution rate (%) | | Taste test | | | |
|---|---|---|---|---|---|---|---|---|
| | | | 30 min | 60 min | Bitterne ss level of API | Bitterness level of coated granules | Percent reduction in bitterness (%) | Gritty feeling |
| F706-1 | 4.84 | 8.77 | 102.1 | 102.2 | 4 | 1.92 | 52.0 | 0.58 |

### 3. Other test data:

### (1) Particle size distribution

**Table 74**

| Batch No. | Particle size test | | |
|---|---|---|---|
| | Test results/µm | Average/µm | Test method |
| F706-1 | Dv(10) | 247 | Dry particle size |
| | Dv(50) | 358 | |
| | Dv(90) | 502 | |

### (2) Scanning electron microscope

The wet-granulated fluidized-coated granules have a scanning electron microscope appearance image as shown in FIG. 15, and a section image as shown in FIG. 16.

The test data show that the vonoprazan fumarate granules prepared by using the wet granulation-fluidized coating process have a not-rounded appearance, large particle size, a Dv(50) reaching 358 µm, a Dv(90) reaching 502 µm, a reduction in bitterness in a taste test by 52%, and a gritty feeling of 0.58. The taste-masking effect is not ideal, and there is a gritty feeling.

### Comparative example 6

### Spray loading-fluidized coating micropellet preparation process

### 1. Prescription composition:

**Table 75**

| Materia l name | Fumaric acid Vonopra zan | Microcr ystalline cellulos e | Hydrox ypropyl cellulos e | Kollicoat^{®} 100 P | Fumari c acid | Butylated hydroxyto luene | Tributyl acetylcitr ate | Talc powd er |
|---|---|---|---|---|---|---|---|---|
| Functio n | Active ingredie nt | Pellet core matrix excipien t | Binder | Isolating layer | Neutral izer | Antioxida nt | Plasticize r | Anti-sticki ng agent |
| Prescrip tion percent age (%) | 6.54 | 61.23 | 4.90 | 19.59 | 0.39 | 0.49 | 2.94 | 3.92 |
| Total weight per formulation unit (g) 0.35 | | | | | | | | |

2. Preparation procedures: blank core - fluidized medicament loading - spray coating - drying - obtaining coated micropellets.

The technical specifications of the micropellets are shown in Table 76.

**Table 76**

| Material name | Material weight gain percentage content (%) | Moisture content (%) |
|---|---|---|
| Loaded micropellet (with core) | 18% (10% weight gain for main medicament) | 1.50% |
| Finished micropellet | 5% | 0.91% |
| Yield (%) | 76% | |

### 3. Product testing

**Table 77**

| Test item | Conte nt (%) | In vitro release rate (%) | pH 1.0 medium dissolution rate (%) | | Taste test | | | |
|---|---|---|---|---|---|---|---|---|
| | | | 30 min | 60 min | Bitterne ss level of API | Bitterness level of coated pellet | Percent reduction in bitterness (%) | Gritt y feelin g |
| F706-2 | 7.2 | 2.49 | 75.2 | 95.5 | 4 | 1.50 | 62.5 | 1 |

### 4. Other test data:

### (1) Particle size distribution

**Table 78**

| Batch No. | | Particle size test | |
|---|---|---|---|
| | Test results/µm | Average/µm | Test method |
| F706-2 | Dv(10) | 215 | Dry particle size |
| | Dv(50) | 349 | |
| | Dv(90) | 522 | |

### (2) Scanning electron microscope

The spray-loaded fluidized-coated micropellets have a scanning electron microscope appearance image as shown in FIG. 17, and a section image as shown in FIG. 18.

The test data show that the vonoprazan fumarate micropellets prepared by using the spray-loading fluidized-coating process have a relatively rounded appearance, large particle size, a Dv(50) reaching 349 µm, a Dv(90) reaching 522 µm, a reduction in bitterness in a taste test by 62.5%, and a gritty feeling of 1. The taste-masking effect is not ideal, and there is a noticeable gritty feeling.

The detailed description above is a specific description of one of the realizable examples of the present disclosure, which is not intended to limit the patent scope of the present disclosure, and any equivalent implementation or modification made without departing from the present disclosure should be included within the scope of the technical solutions of the present disclosure.

## Claims

1. A taste-masking microsphere, comprising the following components in parts by weight: 1.5 to 21.2 parts of a medicament ingredient, 35 to 110 parts of a medicament carrier, 0 to 30 parts of an alkalizing agent, and 0 to 8 parts of a plasticizer.

2. The taste-masking microsphere according to claim 1, wherein the taste-masking microsphere comprises the following components in parts by weight: 5 to 10 parts of the medicament ingredient, 50 to 90 parts of the medicament carrier, 0 to 8 parts of the alkalizing agent, and 2 to 8 parts of the plasticizer.

3. The taste-masking microsphere according to claim 1, wherein the taste-masking microsphere further comprises at least one of an opacifier and an auxiliary agent, wherein the opacifier comprises at least one of zinc oxide and titanium dioxide, and the auxiliary agent comprises at least one of fumaric acid, succinic acid, dibutyl hydroxytoluene, magnesium stearate, silica, talc powder, and povidone.

4. The taste-masking microsphere according to claim 1, wherein the medicament carrier comprises a carrier A and a carrier B, wherein the carrier A is a pH-dependent carrier or a pH-independent carrier and is in an amount of 5 to 40 parts, and the carrier B is a matrix-type carrier in an amount of 30 to 70 parts.

5. The taste-masking microsphere according to claim 4, wherein the amount of the carrier A is 10 to 30 parts; and the amount of the carrier B is 30 to 60 parts.

6. The taste-masking microsphere according to claim 4, wherein the carrier A comprises at least one of a polyacrylic resin, methyl methacrylate-diethylaminoethyl methacrylate copolymer, and a polyvinyl acetal diethylaminoacetate; and the matrix-type carrier comprises at least one of ethylcellulose, microcrystalline cellulose, sodium carboxymethyl cellulose, cellulose acetate, polyvinyl acetate, ammonio methacrylate copolymer type A, ammonio methacrylate copolymer type B, crospovidone, and maltodextrin.

7. The taste-masking microsphere according to claim 6, wherein the polyacrylic resin comprises at least one of Eudragit EPO, Eudragit E100, Eudragit RLPO, Eudragit RL100, Eudragit RSPO, and Eudragit RS100, the methyl methacrylate-diethylaminoethyl methacrylate copolymer is Kollicoat Smartseal 30D100P, and the ethylcellulose comprises at least one of N7, N10, N22, and N50.

8. The taste-masking microsphere according to claim 7, wherein the pH-dependent carrier comprises at least one of Eudragit EPO and Eudragit E100; the pH-independent carrier comprises at least one of Eudragit RLPO and Eudragit RSPO, preferably a mixture of Eudragit RLPO and Eudragit RSPO in a mass ratio of 1 : 1 to 8 : 1; and the matrix-type carrier comprises at least one of N7 and N10.

9. The taste-masking microsphere according to claim 1, wherein the alkalizing agent comprises at least one of sodium carbonate, sodium bicarbonate, magnesium oxide, meglumine, and tromethamine; and the plasticizer comprises at least one of diethyl phthalate, tributyl citrate, polyethylene glycol 6000, and triethyl citrate.

10. The taste-masking microsphere according to claim 9, wherein the alkalizing agent comprises at least one of sodium bicarbonate and magnesium oxide; and the plasticizer is triethyl citrate.

11. The taste-masking microsphere according to claim 1, wherein the medicament ingredient is a compound with a bitter taste or abnormal flavor, preferably an alkaloid compound, more preferably at least one of an organic amine alkaloid and a nitrogen-containing heterocyclic alkaloid, and further preferably comprises at least one of vonoprazan, a pharmaceutically acceptable salt of vonoprazan, berberine hydrochloride, sildenafil citrate, azithromycin, cefuroxime axetil, vardenafil hydrochloride, metformin hydrochloride, paroxetine hydrochloride, sitagliptin hydrochloride, sertraline hydrochloride, pyridostigmine bromide, lidocaine hydrochloride, famotidine, ibuprofen, tramadol hydrochloride, allitride, bepotastine besilate, acetaminophen, colchicine, racecadotril, fluoxetine hydrochloride, flucloxacillin sodium, lacosamide, clarithromycin, donepezil hydrochloride, rivaroxaban, linezolid, cefcapene pivoxil, cefetamet pivoxil, meropenem, moxifloxacin, fluvoxamine maleate, rebamipide, cefteram pivoxil, verapamil, quetiapine, amisulpride, sulpiride, metoprolol, pravastatin, atomoxetine, escitalopram, tilidine hydrochloride, ondansetron, vortioxetine hydrobromide, domperidone, zopiclone, roxatidine, loperamide, diphenhydramine, epinastine, mirabegron, solifenacin, irbesartan, vortioxetine hydrobromide, ticagrelor, captopril, colesevelam hydrochloride, rasagiline, nintedanib esylate, enalapril maleate, apremilast, piroxicam, flutamide, varenicline tartrate, pazopanib, pramipexole, ripretinib, tamsulosin hydrochloride, risperidone, linagliptin, teneligliptin, dabigatran etexilate, odevixibat, micafungin, aprepitant, roflumilast, cefprozil, cefpodoxime proxetil, and tenapanor hydrochloride; and the pharmaceutically acceptable salt of vonoprazan is preferably a fumarate, L-malate, succinate, hemi-L tartrate, dihydrogenphosphate, disulfate, sulfate, hydrochloride, mesylate, phosphate, acetate, citrate, maleate, tartrate, bitartrate, or hydrobromide of vonoprazan.

12. The taste-masking microsphere according to claim 3, wherein when the medicament ingredient is vonoprazan fumarate, the auxiliary agent comprises at least one of fumaric acid, succinic acid, and dibutyl hydroxytoluene, wherein the fumaric acid is added in an amount of 0.08 to 12.42% relative to the total mass of the components of the taste-masking microsphere, the succinic acid is added in an amount of 0.08 to 2% relative to the total mass of the components of the taste-masking microsphere, and the dibutyl hydroxytoluene is added in an amount of 0.08 to 1% relative to the total mass of the components of the taste-masking microsphere.

13. The taste-masking microsphere according to claim 12, wherein the fumaric acid is added in an amount of 0.08 to 10% relative to the total mass of the components of the taste-masking microsphere.

14. A process for preparing the taste-masking microsphere according to any one of claims 1-13, comprising the following steps:
(1) preparing a dispersed phase of microspheres: dissolving the components of the taste-masking microspheres in a solvent A as a dispersed phase of microspheres;
(2) preparing a continuous phase for microspheres: selecting a solvent B as the continuous phase for microspheres; and
(3) pouring the continuous phase for microspheres into a reactor, then feeding the dispersed phase of microspheres thereto from the bottom of the reactor, forming spheres by dispersion, followed by solidifying, filtering, washing, and drying of the spheres to obtain the taste-masking microspheres.

15. The process according to claim 14, wherein the solvent A in step (1) is a mixed solvent of an organic solvent C and water, the mass percentage of water in the solvent A is 0 to 35%, and the organic solvent C comprises at least one of methanol, ethanol, isopropanol, 1,2-propanediol, acetone, tetrahydrofuran, methyltetrahydrofuran, toluene, xylene, acetonitrile, N,N-dimethylacetamide, ethyl acetate, n-butanol, dichloromethane, trichloromethane, tetrachloroethane, and methylpropylene glycol acetate.

16. The process according to claim 15, wherein in step (1), the mass percentage of water in the solvent A is 0 to 33.5%, preferably 0 to 10.5%, and the solvent C comprises at least one of methanol, ethanol, isopropanol, acetone, ethyl acetate, n-butanol, and dichloromethane.

17. The process according to claim 14, wherein in step (1), the solvent C is a mixed solvent of two solvents, specifically comprising any one of the following mixed solvents: a) a mixed solvent of ethyl acetate and ethanol in a volume ratio of 4 : 1 to 1 : 1, preferably 1.5 : 1 if the medicament carrier comprises a pH-dependent carrier, or in a volume ratio of 25 : 1 to 5 : 1, preferably 20 : 1 if the medicament carrier comprises a pH-independent carrier;
b) a mixed solvent of n-butanol and ethanol in a volume ratio of 3 : 1 to 1.2 : 1, preferably 1.5 : 1;
c) a mixed solvent of dichloromethane and ethanol in a volume ratio of 1.7 : 1 to 1 : 1, preferably 1.5 : 1;
d) a mixed solvent of ethyl acetate and methanol in a volume ratio of 3 : 1 to 1.15 : 1, preferably 1.5 : 1;
e) a mixed solvent of ethyl acetate and isopropanol in a volume ratio of 4 : 1 to 1.2 : 1, preferably 1.5 : 1;
f) a mixed solvent of ethyl acetate and acetone in a volume ratio of 2 : 1 to 1 : 1.2, preferably 1.5 : 1; and
g) a mixed solvent of ethyl acetate/acetonitrile in a volume ratio of 2 : 1 to 1 : 1, preferably 1.5 : 1.

18. The process according to claim 14, wherein in step (1), the solvent C is a mixed solvent of three solvents, specifically comprising any one of the following mixed solvents:
(a) a mixed solvent of ethyl acetate, ethanol, and n-butanol at volume percentages of: 60% of ethyl acetate, 30-10% of ethanol, and 10-30% of n-butanol; preferably 60% of ethyl acetate, 20% of ethanol, and 20% of n-butanol;
(b) a mixed solvent of ethyl acetate, ethanol, and methanol at volume percentages of: 60% of ethyl acetate, 30-10% of ethanol, and 10-30% of methanol; preferably 60% of ethyl acetate, 20% of ethanol, and 20% of methanol;
(c) a mixed solvent of ethyl acetate, ethanol, and isopropanol at volume percentages of: 60% of ethyl acetate, 30-10% of ethanol, and 10-30% of isopropanol; preferably 60% of ethyl acetate, 20% of ethanol, and 20% of isopropanol; and
(d) a mixed solvent of ethyl acetate, ethanol, and acetone at volume percentages of: 60% of ethyl acetate, 30-10% of ethanol, and 10-30% of acetone; preferably 60% of ethyl acetate, 20% of ethanol, and 20% of acetone.

19. The process according to claim 14, wherein the solvent B in step (2) comprises water or a mixed solvent of water and at least one of ethyl acetate, ethanol, liquid paraffin and dichloromethane.

20. The process according to claim 19, wherein the solvent B in step (2) is water.

21. The process according to claim 19 or 20, wherein the continuous phase for microspheres in step (2) further comprises a surfactant, and the amount of the surfactant in the continuous phase for microspheres is 0.01 to 2 wt%, preferably 0.05 to 0.8 wt%.

22. The process according to claim 21, wherein the surfactant comprises at least one of sodium oleate, Tween, polyvinyl alcohol, sodium dodecyl sulfate, and sodium carboxymethyl cellulose.

23. The process according to claim 21, wherein when the surfactant is sodium oleate, the mass percentage of sodium oleate in the continuous phase for microspheres is 0.01 to 0.05%;
when the surfactant is Tween 80, the mass percentage of Tween 80 in the continuous phase for microspheres is 0.02 to 2%;
when the surfactant is polyvinyl alcohol, the polyvinyl alcohol comprises at least one of 03-88, 05-88, 17-88, 20-88 and 25-88, and the mass percentage of the polyvinyl alcohol in the continuous phase for microspheres is 0.05 to 2.0%, preferably 0.2 to 0.4%;
when the surfactant is sodium dodecyl sulfate, the mass percentage of sodium dodecyl sulfate in the continuous phase for microspheres is 0.02 to 0.2%, preferably 0.05%; and
when the surfactant is sodium carboxymethyl cellulose, the mass percentage of sodium carboxymethyl cellulose in the continuous phase for microspheres is 0.02 to 0.25%, preferably 0.05%.

24. The process according to claim 14, wherein the continuous phase for microspheres in step (2) is at a temperature of 2-30°C and a pH of 6-11.

25. The process according to claim 24, wherein the continuous phase for microspheres in step (2) is at a temperature of 2-10°C and a pH of 6-8.

26. The process according to claim 14, wherein the mass ratio of the continuous phase for microspheres to the dispersed phase of microspheres is (14.7 to 123.5) : 1, and the ratio of the total mass of the components of the taste-masking microspheres to the mass of the solvent A is (6.5 to 21.5) : 100.

27. The process according to claim 26, wherein the mass ratio of the continuous phase for microspheres to the dispersed phase of microspheres is (20 to 50) : 1, and the ratio of the total mass of the components of the taste-masking microspheres to the mass of the solvent A is (6.5 to 15) : 100.

28. The process according to claim 14, wherein the reactor in step (3) is in the shape of an inverted triangular pyramid frustum, with feed ports provided at the bottom of the reactor.

29. The process according to claim 28, wherein the cross section of the inverted triangular pyramid frustum is an isosceles triangle.

30. The process according to claim 29, wherein the isosceles triangle has a base angle β of 45° - 75°, preferably 60°; the height of the reactor is 10 cm - 200 cm; and the included angle α between a side plane edge and the bottom face of the inverted triangular pyramid frustum is 30° ≤ α < 90°, preferably 60° ≤ α < 90°, further preferably α = 79° - 85°.

31. The process according to claim 14, wherein 2-4 layers of stirring blades are provided in the reactor, and the blades are a push-down stirring paddles providing stirring at a rotational speed of 50-900 rpm; wherein the filtrate obtained after the filtering in step (3) is reused in the next reaction with a reuse proportion of 10-80%; or the solvents in the filtrate obtained after the filtering in step (3) are recovered first, and then the recovered solvents are used for the preparation of the solvent A or solvent B.

32. A composition, comprising the taste-masking microsphere according to claim 12 or claim 13.

33. The composition according to claim 32, wherein the composition comprises, in parts by weight, 10-20 parts of the taste-masking microsphere, 500-1,500 parts of amoxicillin, and 50-150 parts of rifabutin.

34. The composition according to claim 33, wherein the mass ratio of the taste-masking microsphere, amoxicillin, and rifabutin is 20 : 1000 : 50, 20 : 1000 : 75, 20 : 1000 : 100, 20 : 1000 : 125, or 20 : 1000 : 150.

35. The composition according to claim 33, wherein the amoxicillin and/or rifabutin is in the form of granules, micropellets, or microspheres.

36. Use of the composition according to any one of claims 32-35 in the preparation of a medicament against diseases related to *Helicobacter pylori* infection.

37. A formulation comprising the taste-masking microsphere according to any one of claims 1-13, or a taste-masking microsphere obtained by the process according to any one of claims 14-31, or the composition according to any one of clams 32-35.

38. The formulation according to claim 37, wherein the dosage form of the formulation is a dry suspension, a film, a granule, a capsule, or a tablet.
